# EUROPEAN PATENT APPLICATION

(11) **EP 4 843 579 A1**
(43) Date of publication of application: **25.11.2026**
(21) Application number: 25760668.1
(22) Date of filing: 15.01.2025
(51) Int. Cl.: A61K 36/28, C12N 5/04, C12N 15/82, C12Q 1/68, A61P 9/10, A61P 1/16

(54) **SAUSSUREA INVOLUCRATA CELL CULTURE AND USE THEREOF**

(30) Priority: 26.02.2024 CN 202410209155
(71) Applicant: Dalian Practical Biotechnology Co., Ltd., Dalian, Liaoning 116001 (CN)
(72) Inventor: LIU, Hanshi, Dalian, Liaoning 116001 (CN); LIU, Yu, Dalian, Liaoning 116001 (CN); CUI, Xiaolin, Dalian, Liaoning 116001 (CN); TANG, Junnan, Dalian, Liaoning 116001 (CN)
(74) Representative: Wang, Bo
(86) International application number: PCT/CN2025/072430
(87) International publication number: WO 2025/180124

(57) **Abstract**

A composition derived from a callus culture of snow lotus cells and a use thereof. The composition includes: a) one or more flavonoid compounds in an amount of 7 wt%-20 wt%; b) one or more phenylpropanoid derivatives in an amount of 2.1 wt%-14.1 wt%; c) a carboxylic acid and one or more derivatives thereof, the carboxylic acid and one or more derivatives thereof including dicaffeoylquinic acid in an amount of 1.6 wt%-35 wt%; and d) other components.

## Description

### CROSS-REFERENCE TO RELATED APPLICATIONS

This application claims priority to Chinese Application No. 202410209155.X, filed on February 26, 2024, the entire contents of each of which are incorporated herein by reference.

### TECHNICAL FIELD

The present disclosure relates to the technical field of biomedicine, and in particular to a composition derived from a callus culture of snow lotus cells and use thereof.

### BACKGROUND

*Saussurea involucrata* Kar. et Kir., also known as "snow lotus", is a perennial herb belonging to the genus *Saussurea* in the family Asteraceae, and a rare and precious Chinese herbal medicine endemic to Xinjiang, China. There are 12 species and 1 variety of snow lotus plants commonly used as crude drugs. To date, only *Saussurea involucrata* is included in the Chinese Pharmacopoeia (2005 edition). In traditional Chinese medicine, *Saussurea involucrata* is considered to possess the effects of dispelling cold and removing dampness, activating blood circulation and unblocking meridians, and balancing Yin and Yang. Modern scientific research has demonstrated that *Saussurea involucrata* mainly contains flavonoids and lignans, as well as biologically active compounds, such as coumarins, sesquiterpenes, phenolic aldehydes, phenylpropanols, sterols, polysaccharides, and ceramides, thereby exhibiting therapeutic effects against tumors, leukemia, and various cancers and inflammatory conditions, conferring significant medicinal value. However, due to its specific growth environment, slow growth rate, and difficulties in artificial cultivation, the long-term predatory excavation has endangered the wild resources of *Saussurea involucrata.*

Plant cell culture technology is a technology for culturing and propagating plant cells, tissues, or organs in an environment without living organisms, which is an effective approach for solving the shortage of medicinal plant resources. Using plant cell culture technology is a significant path for sustainable development to solve the resource problem of natural Snow Lotus. Therefore, the plant cell culture technology for *Saussurea involucrata* and a composition derived from a callus culture of snow lotus cells have great application potential.

Atherosclerosis (AS) is a disease that seriously endangers human health and is the most common pathogenic cause of coronary thrombosis and ischemic stroke. AS is characterized in that lesions start from the intima of an affected artery and gradually cause lipid accumulation, fibrous tissue proliferation, and calcium deposition, and even lead to calcification of the arterial media. Based on this, intraplaque hemorrhage, plaque rupture, and local thrombosis occur, which are a primary etiology of the majority of cardiovascular diseases. Therefore, effective prevention and treatment of AS is a key step in the prevention of cardiovascular and cerebrovascular diseases. The research and development of lipid-lowering and anti-inflammatory drugs is of great importance for the prevention and treatment of AS.

Chronic long-term liver injury is a chronic inflammatory disease of the liver caused by different predisposing factors, such as drug-induced hepatitis, viral infection, long-term excessive alcohol intake, fatty liver, and immune reactions. Drug-induced injury and viral injury are the main pathogenic causes of chronic long-term liver injury. Chronic long-term liver injury is often accompanied by inflammatory responses, which can lead to hepatocyte injury and apoptosis, as well as excessive release of transaminase into the blood, thereby affecting normal hepatic function. Therefore, the development of appropriate pharmaceutical agents for the prevention and treatment of chronic long-term liver injury is of great importance.

Therefore, it is necessary to provide a composition derived from a callus culture of snow lotus cells that can replace plant-derived *Saussurea involucrata* and can be prepared by the plant cell culture technique, and to provide a use of the composition derived from the callus culture of snow lotus cells in the preparation of a medicament for a specific disease/indication.

### SUMMARY

One or more embodiments of the present disclosure provide a composition derived from a callus culture of snow lotus cells. The composition comprises: a) one or more flavonoid compounds in an amount of 7 wt%-20 wt%; b) one or more phenylpropanoid derivatives in an amount of 2.1 wt%-14.1 wt%; c) a carboxylic acid and one or more derivatives thereof, wherein the carboxylic acid and one or more derivatives thereof comprise dicaffeoylquinic acid in an amount of 1.6 wt%-35 wt%; and d) other components.

In some embodiments, the one or more phenylpropanoid derivatives comprise syringin in an amount of 0.3 wt%-5 wt% and chlorogenic acid in an amount of 0.3 wt%-1.3 wt%.

In some embodiments, the dicaffeoylquinic acid comprises 1,5-dicaffeoylquinic acid in an amount 1.5 wt%-7.7 wt%, and a proportion of the 1,5-dicaffeoylquinic acid in the dicaffeoylquinic acid is 22%-95%.

In some embodiments, the High Performance Liquid Chromatography (HPLC) characteristic chromatogram of the composition derived from the callus culture of snow lotus cells at 265 nm comprises 9 characteristic peaks; wherein peak 1 is syringin, peak 2 is chlorogenic acid, peak 4 is 1,4-dicaffeoylquinic acid, peak 5 is 1,5-dicaffeoylquinic acid, peak 6 is 3,5-dicaffeoylquinic acid, peak 7 is dicaffeoylquinic acid malate ester, peak 8 is 1,3,5-tricaffeoylquinic acid malate ester, and peak 9 is 1,3,5-tricaffeoylquinic acid methyl malate ester.

In some embodiments, in the HPLC characteristic chromatogram of the composition at 265 nm, with peak 5 as a reference peak, a relative retention time of each characteristic peak is within a range of ±5% of a specified value as follows: a specified value for peak 1 is 0.369; a specified value for peak 2 is 0.450; a specified value for peak 3 is 0.668; a specified value for peak 4 is 0.918; a specified value for peak 5 is 1.000; a specified value for peak 6 is 1.057; a specified value for peak 7 is 1.140; a specified value for peak 8 is 1.622; and a specified value for peak 9 is 1.645.

One or more embodiments of the present disclosure also provide a method for producing a composition derived from a callus culture of snow lotus cells. The method comprises: obtaining a differentiated embryogenic pluripotent stem cell population derived from wild *Saussurea involucrata*; and performing amplification culture on the differentiated embryogenic pluripotent stem cell population to harvest a culture broth containing the composition; wherein the differentiated embryogenic pluripotent stem cell population is obtained by a process of controlled differentiation, and the process causes the differentiated embryogenic pluripotent stem cell population to possess the following characteristics: using *in vitro* cells of the wild *Saussurea involucrata* as a control group, differentiation-related genes in the differentiated embryogenic pluripotent stem cell population are expressed at a reduced level, and the differentiation-related genes comprise WOX4, LBD18, WOX5, and PLT4.

In some embodiments, the composition comprises: a) one or more flavonoid compounds in an amount of 7 wt%-20 wt%; b) one or more phenylpropanoid derivatives in an amount of 2.1 wt%-14.1 wt%; c) a carboxylic acid and one or more derivatives thereof, wherein the carboxylic acid and one or more derivatives thereof comprise dicaffeoylquinic acid in an amount of 6.8 wt%-8.1 wt%; and d) other components.

In some embodiments, the dicaffeoylquinic acid comprises 1,5-dicaffeoylquinic acid in an amount of 1.5 wt%-7.7 wt%, and a proportion of the 1,5-dicaffeoylquinic acid in the dicaffeoylquinic acid is 22%-95%.

In some embodiments, expression levels of the WOX4 and the LBD18 in the differentiated embryogenic pluripotent stem cell population are less than 1/10 of expression levels of corresponding genes in the control group; and expression levels of the WOX5 and the PLT4 in the differentiated embryogenic pluripotent stem cell population are less than 1/2 of expression levels of corresponding genes in the control group.

In some embodiments, the method further comprises: during the amplification culture, performing metabolic regulation on the differentiated embryogenic pluripotent stem cell population based on flavonoid metabolic pathway enhancement.

In some embodiments, the metabolic regulation causes the differentiated embryogenic pluripotent stem cell population to possess the following characteristics: flavonoid metabolic pathway-related genes in the differentiated embryogenic pluripotent stem cell population are expressed, and the flavonoid metabolic pathway-related genes comprise PAL, C4H, 4CL, CHI, C3H, F5H, and CAD; and using the *in vitro* cells of the wild *Saussurea involucrata* as the control group, expressions of the C3H, the F5H, and the CAD in the differentiated embryogenic pluripotent stem cell population are increased.

In some embodiments, the method further comprises separating the composition from the culture broth, and preparing an extract from the composition; the extract is prepared by a process including: subjecting the composition to repeated freeze-thawing to obtain a thawed mixture; performing an extraction treatment on the thawed mixture using an aqueous ethanol solution to obtain a preliminary extract, wherein the extraction treatment includes at least one of ultrasonic extraction and stirring extraction; separating a solid part and a liquid part of the preliminary extract; and performing a drying process on the liquid part to obtain the extract.

Some embodiments of the present disclosure also provide a use of the composition derived from the callus culture of snow lotus cells as described above in the preparation of a medicament for AS.

In some embodiments, compared to a model group with AS, the formation of atherosclerotic plaques in blood vessels is reduced in a treatment group administered with the composition.

In some embodiments, compared to a model group with AS, gut microbiota distribution is regulated in a treatment group administered with the composition, and the regulation reduces activation and migration of macrophages, and reduces apoptosis of endothelial cells, thereby reducing the formation of atherosclerotic plaques.

In some embodiments, the composition is obtained by performing amplification culture on a differentiated embryogenic pluripotent stem cell population derived from wild *Saussurea involucrata*, and the differentiated embryogenic pluripotent stem cell population is obtained through a process of controlled differentiation, wherein the process causes the differentiated embryogenic pluripotent stem cell population to possess the following characteristic: using *in vitro* cells of wild *Saussurea involucrata* as a control, differentiation-related genes of the differentiated embryogenic pluripotent stem cell population are expressed at a reduced level, and the differentiation-related genes comprise WOX4, LBD18, WOX5, and PLT4.

One or more embodiments of the present disclosure also provide a use of the composition derived from the callus culture of snow lotus cells as described above in the preparation of a medicament for chronic long-term liver injury.

In some embodiments, compared to a model group with chronic long-term liver injury, levels of liver function injury-related indicators are reduced in a treatment group administered with the composition, and the liver function injury-related indicators comprise ALT, AST, GGT, and ALP; and in the treatment group, generation of fibrous tissue in liver tissue is reduced, and lipid droplet formation and lipid deposition are reduced, thereby reducing the formation of alcoholic fatty liver.

In some embodiments, compared with a model group with chronic long-term liver injury, in a treatment group administered with the composition, gut microbiota distribution is regulated, wherein the regulation increases a quantity of beneficial bacteria and decreases a quantity of harmful bacteria, thereby reducing a difference between the gut microbiota distribution of the treatment group and gut microbiota distribution of a normal group.

In some embodiments, the composition is obtained by performing amplification culture on a differentiated embryogenic pluripotent stem cell population derived from wild *Saussurea involucrata*, and the differentiated embryogenic pluripotent stem cell population is obtained through a process of controlled differentiation, wherein the process causes the differentiated embryogenic pluripotent stem cell population to possess the following characteristic: using *in vitro* cells of wild *Saussurea involucrata* as a control, differentiation-related genes of the differentiated embryogenic pluripotent stem cell population are expressed at a reduced level, and the differentiation-related genes comprise WOX4, LBD18, WOX5, and PLT4.

### BRIEF DESCRIPTION OF THE DRAWINGS

The present disclosure will be further described by way of exemplary embodiments, which will be described in detail by means of the accompanying drawings. These embodiments are not restrictive, and in these embodiments, the same numbering denotes the same structure, wherein:
FIG. 1 is an interval bar chart illustrating relative expression levels of differentiation-related genes of an embryogenic pluripotent stem cell population at the mRNA level according to some embodiments of the present disclosure;
FIG. 2 is an interval bar chart illustrating relative expression levels of key enzymes in the flavonoid metabolic pathway at the mRNA level according to some embodiments of the present disclosure;
FIG. 3 is a chromatographic comparison chart of characteristic peaks of a composition derived from a callus culture of snow lotus cells and *Saussurea involucrata* at 265 nm according to some embodiments of the present disclosure;
FIG. 4A is an ultraviolet spectrum of 1,5-dicaffeoylquinic acid according to some embodiments of the present disclosure;
FIG. 4B is an ultraviolet spectrum of characteristic peak 5 of the composition derived from the callus culture of snow lotus cells according to some embodiments of the present disclosure;
FIG. 5A is an ultraviolet spectrum of rutin according to some embodiments of the present disclosure;
FIG. 5B is an ultraviolet spectrum of characteristic peak 6 of the composition derived from the callus culture of snow lotus cells according to some embodiments of the present disclosure;
FIG. 5C is an ultraviolet spectrum of characteristic peak 6 of *Saussurea involucrata* according to some embodiments of the present disclosure;
FIG. 6A is an interval bar chart illustrating cell viability of human umbilical vein endothelial cells (HUVECs) in different groups according to some embodiments of the present disclosure;
FIG. 6B is an interval bar chart illustrating cell apoptosis according to some embodiments of the present disclosure;
FIG. 6C is an interval bar chart illustrating cell necrosis according to some embodiments of the present disclosure;
FIG. 6D is an interval bar chart illustrating cell mechanical damage according to some embodiments of the present disclosure;
FIG. 6E is an interval bar chart illustrating fluorescence intensity of HUVECs in different groups based on terminal deoxynucleotidyl transferase dUTP nick end labeling (TUNEL) staining according to some embodiments of the present disclosure;
FIG. 6F is a fluorescence image of HUVECs in different groups based on TUNEL/4',6-diamidino-2-phenylindole (DAPI) double staining, obtained by a laser confocal microscope according to some embodiments of the present disclosure;
FIG. 6G is an interval bar chart illustrating fluorescence intensity from quantitative analysis of reactive oxygen species (ROS) production by HUVECs in different groups using flow cytometry according to some embodiments of the present disclosure;
FIG. 6H is a fluorescence image of HUVECs in different groups based on 2',7'-dichlorodihydrofluorescein diacetate (DCFH-DA)/DAPI double staining, obtained by a laser confocal microscope according to some embodiments of the present disclosure;
FIG. 6I is an interval bar chart illustrating fluorescence intensity of HUVECs in different groups based on DCFH-DA staining according to some embodiments of the present disclosure;
FIG. 7A is a bar chart illustrating a gene expression level of tumor necrosis factor-α (TNF-α) in RAW264.7 cells in different groups according to some embodiments of the present disclosure;
FIG. 7B is a bar chart illustrating a gene expression level of interleukin-6 (IL-6) in RAW264.7 cells in different groups according to some embodiments of the present disclosure;
FIG. 7C is a bar chart illustrating a gene expression level of interleukin-10 (IL-10) in RAW264.7 cells in different groups according to some embodiments of the present disclosure;
FIG. 7D is a bar chart illustrating a gene expression level of interleukin-1β (IL-1β) in RAW264.7 cells in different groups according to some embodiments of the present disclosure;
FIGs. 7E-7G are images of Oil Red O-stained RAW264.7 cells in different groups according to some embodiments of the present disclosure;
FIG. 7H is a bar chart illustrating a relative area of lipid droplets in RAW264.7 cells in different groups according to some embodiments of the present disclosure;
FIG. 8A is an interval bar chart illustrating a concentration of a key factor, glucose (GLU), in blood of mice in different groups according to some embodiments of the present disclosure;
FIG. 8B is an interval bar chart illustrating a concentration of a key factor cholesterol (CHOL) in blood of mice in different groups according to some embodiments of the present disclosure;
FIG. 8C is an interval bar chart illustrating a concentration of a key factor, triglycerides (TG), in blood of mice in different groups according to some embodiments of the present disclosure;
FIG. 8D is an interval bar chart illustrating a concentration of a key factor, high-density lipoprotein cholesterol (HDL-C), in blood of mice in different groups according to some embodiments of the present disclosure;
FIG. 8E is an interval bar chart illustrating a concentration of a key factor, low-density lipoprotein cholesterol (LDL-C), in blood of mice in different groups according to some embodiments of the present disclosure;
FIG. 8F is an Oil Red O staining image of thoracic aortas of mice in different groups according to some embodiments of the present disclosure;
FIG. 8G is a staining image of tissue sections of the thoracic aortas of mice in different groups according to some embodiments of the present disclosure;
FIG. 8H is a bar chart of quantitative analysis of Oil Red O staining according to some embodiments of the present disclosure;
FIG. 8I is a bar chart of quantitative analysis of Masson's trichrome staining according to some embodiments of the present disclosure;
FIG. 8J is a bar chart of quantitative analysis of TUNEL staining according to some embodiments of the present disclosure;
FIG. 8K is an immunohistochemical (IHC) staining image of tissue sections of arteries of mice in different groups according to some embodiments of the present disclosure;
FIG. 9A is a chart illustrating community species richness of gut microbiota evaluated by Chao1 richness estimator, Shannon diversity index, and Simpson index according to some embodiments of the present disclosure;
FIG. 9B is a plot of principal coordinate analysis (PCoA) and non-metric multidimensional scaling (NMDS) of the gut microbiota according to some embodiments of the present disclosure;
FIG. 9C is a Venn diagram of the gut microbiota of samples from each group according to some embodiments of the present disclosure;
FIGs. 9D-9E are clustering histograms of the gut microbiota according to some embodiments of the present disclosure;
FIGS. 9F-9G are graphs illustrating relative abundances of different microorganisms at family and genus levels among groups according to some embodiments of the present disclosure;
FIGS. 9H-9I are principal component analysis (PCA) plots of different microorganisms at the family and genus levels among groups according to some embodiments of the present disclosure;
FIG. 10A is a hematoxylin and eosin (HE) staining image of liver sections of mice in different groups according to some embodiments of the present disclosure;
FIG. 10B is an Oil Red O staining image of liver sections of mice in different groups according to some embodiments of the present disclosure;
FIG. 10C is an IHC staining image of liver sections of mice in different groups according to some embodiments of the present disclosure.

### DETAILED DESCRIPTION

It should be understood that, although the terms "first", "second", "third", or the like may be used herein to describe various elements, these elements should not be limited by these terms. These terms are merely used to distinguish one element from another. For example, a first product may be referred to as a second product, and similarly, without departing from the scope of the exemplary embodiments of the present disclosure, the second product may be referred to as the first product.

As used in the present disclosure and the claims, unless the context clearly indicates otherwise, the words "a", "an", "one", and/or "the", or the like are not limited to the singular and may also include the plural. Generally speaking, the terms "comprise" and "include" merely indicate that explicitly identified steps and elements are included, and these steps and elements do not constitute an exclusive enumeration, and a method or a device may also include other steps or elements.

Unless otherwise defined, all technical and scientific terms used herein have the same meaning as commonly understood by one of ordinary skill in the art to which the present disclosure pertains.

Some embodiments of the present disclosure provide a composition derived from a callus culture of snow lotus cells. The composition derived from the callus culture of snow lotus cells comprises: a) one or more flavonoid compounds; b) one or more phenylpropanoid derivatives; c) a carboxylic acid and one or more derivatives thereof; and d) other components.

As used herein, the term "flavonoid compounds" refers to a class of compounds containing an aromatic ring and two benzene rings with a ketone group in the middle.

In some embodiments, rutin is a common flavonoid compound found in plant-derived *Saussurea involucrata*.

In some embodiments, the flavonoid compounds of the composition derived from the callus culture of snow lotus cells comprise one or more of quinic acid, protocatechuic aldehyde, protocatechuic acid, eriodictyol, ferulic acid, syringin, chlorogenic acid, caffeic acid, coumaroylquinic acid, luteolin-7-O-glucoside, eriodictyol-7-O-glucoside, 1,5-O-dicaffeoylquinic acid, 3,5-O-dicaffeoylquinic acid, methyl caffeate, luteolin, and apigenin.

In some embodiments, the amount of the flavonoid compound in the composition derived from the callus culture of snow lotus cells is 7 wt% to 20 wt%. For example, the amount of the flavonoid compound in the composition derived from the callus culture of snow lotus cells may be 7 wt%, 8 wt%, 9 wt%, 10 wt%, 12 wt%, 14 wt%, 16 wt%, 18 wt%, or 20 wt%, or any range defined by any two of these values. As used herein, the term "amount" refers to a mass content or a mass percentage, calculated on a dry weight basis. In some preferred embodiments, the amount of the flavonoid compound in the composition derived from the callus culture of snow lotus cells is 14 wt% to 20 wt%.

The term "phenylpropanoid derivatives" refers to a class of compounds containing one or more C6-C3 units.

In some embodiments, the amount of the phenylpropanoid derivatives in the composition derived from the callus culture of snow lotus cells is 2.1 wt% to 14.1 wt%. For example, the amount of the phenylpropanoid derivatives in the composition derived from the callus culture of snow lotus cells may be 2.1 wt%, 3 wt%, 4 wt%, 5 wt%, 6 wt%, 7 wt%, 8 wt%, 9 wt%, 10 wt%, 12 wt%, 14 wt%, or 14.1 wt%, or a range defined by any two of these values. In some preferred embodiments, the amount of the phenylpropanoid derivatives in the composition derived from the callus culture of snow lotus cells is 6 wt% to 10 wt%.

In some embodiments, the phenylpropanoid derivative of the composition derived from the callus culture of snow lotus cells comprises syringin and chlorogenic acid.

In some embodiments, the amount of syringin in the composition derived from the callus culture of snow lotus cells is 0.3 wt% to 5 wt%. For example, the amount of syringin in the composition derived from the callus culture of snow lotus cells may be 0.3 wt%, 0.6 wt%, 1.0 wt%, 2.0 wt%, 3.0 wt%, 4.0 wt%, or 5.0 wt%, or a range defined by any two of these values. In some preferred embodiments, the amount of syringin in the composition derived from the callus culture of snow lotus cells is 0.5 wt% to 0.9 wt%.

In some embodiments, the amount of chlorogenic acid in the composition derived from the callus culture of snow lotus cells is 0.3 wt% to 1.3 wt%. For example, the amount of chlorogenic acid in the composition derived from the callus culture of snow lotus cells may be 0.3 wt%, 0.4 wt%, 0.5 wt%, 0.6 wt%, 0.7 wt%, 0.8 wt%, 0.9 wt%, 1.0 wt%, 1.1 wt%, 1.2 wt%, or 1.3 wt%, or a range defined by any two of these values. In some preferred embodiments, the amount of chlorogenic acid in the composition derived from the callus culture of snow lotus cells is 0.6 wt% to 1.0 wt%.

The term "carboxylic acid and derivative thereof" refers to a class of compounds containing a carboxyl group (-COOH), and compounds formed by the substitution of a hydroxyl group on the carboxyl group with other atoms or atomic groups.

In some embodiments, the carboxylic acid and derivative thereof of the composition derived from the callus culture of snow lotus cells comprises dicaffeoylquinic acid. In some embodiments, the amount of dicaffeoylquinic acid in the composition derived from the callus culture of snow lotus cells is 1.6 wt% to 35 wt%. For example, the amount of dicaffeoylquinic acid in the composition derived from the callus culture of snow lotus cells may be 1.6 wt%, 2 wt%, 3 wt%, 4 wt%, 5 wt%, 6 wt%, 7 wt%, 8 wt%, 9 wt%, 10 wt%, 11 wt%, 12 wt%, 13 wt%, 14 wt%, 15 wt%, 16 wt%, 17 wt%, 18 wt%, 19 wt%, 20 wt%, 21 wt%, 22 wt%, 23 wt%, 24 wt%, 25 wt%, 26 wt%, 27 wt%, 28 wt%, 29 wt%, 30 wt%, 31 wt%, 32 wt%, 33 wt%, 34 wt%, or 35 wt%, or a range defined by any two of these values. In some preferred embodiments, the amount of dicaffeoylquinic acid in the composition derived from the callus culture of snow lotus cells is 3.2 wt% to 28 wt%.

In some embodiments, the dicaffeoylquinic acid in the composition derived from the callus culture of snow lotus cells comprises 1,5-dicaffeoylquinic acid. In some embodiments, the amount of 1,5-dicaffeoylquinic acid in the composition derived from the callus culture of snow lotus cells is 1.5 wt%-7.7 wt%. For example, the amount of 1,5-dicaffeoylquinic acid in the composition derived from the callus culture of snow lotus cells may be 1.5 wt%, 1.7 wt%, 1.9 wt%, 2.1 wt%, 2.3 wt%, 2.5 wt%, 2.7 wt%, 2.9 wt%, 3.1 wt%, 3.3 wt%, 3.5 wt%, 3.7 wt%, 3.9 wt%, 4.1 wt%, 4.3 wt%, 4.5 wt%, 4.7 wt%, 4.9 wt%, 5.1 wt%, 5.3 wt%, 5.5 wt%, 5.7 wt%, 5.9 wt%, 6.1 wt%, 6.3 wt%, 6.5 wt%, 6.7 wt%, 6.9 wt%, 7.1 wt%, 7.3 wt%, 7.5 wt%, or 7.7 wt%, or a range defined by any two of these values. In some preferred embodiments, the amount of 1,5-dicaffeoylquinic acid in the composition derived from the callus culture of snow lotus cells is 3.0 wt%-6.2 wt%.

In some embodiments, a proportion of the 1,5-dicaffeoylquinic acid in the dicaffeoylquinic acid of the composition derived from the callus culture of snow lotus cells is 22%-95%. For example, the proportion of the 1,5-dicaffeoylquinic acid in the dicaffeoylquinic acid of the composition derived from the callus culture of snow lotus cells may be 22%, 25%, 30%, 35%, 40%, 45%, 50%, 55%, 60%, 65%, 70%, 75%, 80%, 85%, 90%, or 95%, or a range defined by any two of these values. In some preferred embodiments, the proportion of the 1,5-dicaffeoylquinic acid in the dicaffeoylquinic acid of the composition derived from the callus culture of snow lotus cells is 60% or 70%.

In some embodiments, the dicaffeoylquinic acid in the composition derived from the callus culture of snow lotus cells comprises 3,5-dicaffeoylquinic acid and 1,4-dicaffeoylquinic acid.

In some embodiments, the carboxylic acid and the derivative thereof in the composition derived from the callus culture of snow lotus cells further comprises dicaffeoylquinic acid malate ester, 1,3,5-tricaffeoylquinic acid malate ester, and 1,3,5-tricaffeoylquinic acid methyl malate ester.

In some embodiments, the other components in the composition derived from the callus culture of snow lotus cells comprise proteins. In some embodiments, the amount of the proteins in the composition derived from the callus culture of snow lotus cells is 20% to 35%. For example, the amount of the proteins in the composition derived from the callus culture of snow lotus cells may be 20%, 22%, 24%, 26%, 28%, 30%, 32%, 34%, or 35%, or a range defined by any two of these values.

In some embodiments, the other components in the composition derived from the callus culture of snow lotus cells further comprise organic oxides, lipid compounds, and isoprenol compounds.

In some embodiments, the HPLC characteristic chromatogram of the composition derived from the callus culture of snow lotus cells at 265 nm comprises 9 characteristic peaks. Peak 1 is syringin, peak 2 is chlorogenic acid, peak 4 is 1,4-dicaffeoylquinic acid, peak 5 is 1,5-dicaffeoylquinic acid, peak 6 is 3,5-dicaffeoylquinic acid, peak 7 is dicaffeoylquinic acid malate ester, peak 8 is 1,3,5-tricaffeoylquinic acid malate ester, and peak 9 is 1,3,5-tricaffeoylquinic acid methyl malate ester.

In some embodiments, in the HPLC characteristic chromatogram of the composition derived from the callus culture of snow lotus cells at 265 nm, with the peak 5 as a reference peak, a relative retention time of each characteristic peak is within a range of ±5% of a specified value as follows: a specified value for peak 1 is 0.369; a specified value for peak 2 is 0.450; a specified value for peak 3 is 0.668; a specified value for peak 4 is 0.918; a specified value for peak 5 is 1.000; a specified value for peak 6 is 1.057; a specified value for peak 7 is 1.140; a specified value for peak 8 is 1.622; and a specified value for peak 9 is 1.645.

Some embodiments of the present disclosure also provide a method for detecting an HPLC characteristic chromatogram of a composition derived from a callus culture of snow lotus cells. In some embodiments, the detection of an HPLC characteristic chromatogram of a to-be-tested substance comprises step a1 and step a2.

In step a1, preparing a test solution of the composition derived from the callus culture of snow lotus cells, and preparing a reference solution.

In some embodiments, the test solution is a methanol extract of the composition derived from the callus culture of snow lotus cells. In some embodiments, the test solution is prepared by a process including: adding a powder of the composition derived from a callus culture of snow lotus cells to 60% methanol to obtain a mixture; subjecting the mixture to ultrasonic treatment, followed by cooling to room temperature; replenishing the lost weight with 60% methanol; filtering through a 0.45 µm microporous membrane; and obtaining the test solution. In some embodiments, a weight of the powder of the composition derived from the callus culture of snow lotus cells is approximately 0.2 g, and a volume of the methanol is 25 mL.

In some embodiments, the reference solution is a methanol solution of 1,5-dicaffeoylquinic acid. In some embodiments, the reference solution is prepared using 60% methanol and a reference standard of 1,5-dicaffeoylquinic acid, and each 1 mL of the reference solution comprises 0.3 mg of the reference standard of 1,5-dicaffeoylquinic acid.

In step a2, under predetermined chromatographic conditions, injecting a predetermined amount of the test solution and the reference solution into a liquid chromatograph for measurement, to generate an HPLC characteristic chromatogram.

In some embodiments, the predetermined chromatographic conditions comprise: using octadecylsilane bonded silica gel as a packing material; using methanol as mobile phase A and a 0.3% aqueous formic acid solution as mobile phase B; a flow rate of 0.9 mL/min, and a detection wavelength of 265 nm; and the gradient elution conditions are as follows:
From 0.01 to 15 min, mobile phase A: 20%, mobile phase B: 80%;
From 15 to 25 min, mobile phase A: 20% to 36%, mobile phase B: 80% to 64%;
From 25 to 60 min, mobile phase A: 36% to 37%, mobile phase B: 64% to 63%;
From 60 to 85 min, mobile phase A: 37% to 70%, mobile phase B: 63% to 30%;
From 85 to 90 min, mobile phase A: 70% to 100%, mobile phase B: 30% to 0%;
From 90 to 100 min, mobile phase A: 100%, mobile phase B: 0%;
From 100 to 100.01 min, mobile phase A: 100% to 20%, mobile phase B: 0% to 80%;
At 115 min, stop.

In some embodiments, an injection volume of the test solution and the reference solution is 5 µL.

Some embodiments of the present disclosure also provide a method for quality control of a composition derived from a callus culture of snow lotus cells. The method comprises comparing the HPLC characteristic chromatogram of a to-be-tested substance with the HPLC characteristic chromatogram of the composition derived from the callus culture of snow lotus cells.

It should be understood that the method for detecting the HPLC characteristic chromatogram of the to-be-tested substance is the same as the previously described method for detecting the HPLC characteristic chromatogram of the composition derived from the callus culture of snow lotus cells, and the chromatographic characteristics of the HPLC characteristic chromatogram of the composition derived from the callus culture of snow lotus cells are the same as the chromatographic characteristics of the HPLC characteristic chromatogram of the composition derived from the callus culture of snow lotus cells at 265 nm as described above, and thus further details are not repeated here.

Some embodiments of the present disclosure also provide a method for producing a composition derived from a callus culture of snow lotus cells. The method comprises step b1 and step b2.

In step b1, obtaining a differentiated embryogenic pluripotent stem cell population derived from wild *Saussurea involucrata*.

In some embodiments, the differentiated embryogenic pluripotent stem cell population may be obtained by a process of controlled differentiation. The process causes the differentiated embryogenic pluripotent stem cell population to possess the following features: using *in vitro* cells of the wild *Saussurea involucrata* as a control group, differentiation-related genes in the differentiated embryogenic pluripotent stem cell population are expressed at a reduced level, and the differentiation-related genes comprise WOX4 (NCBI Gene ID: 841113), LBD18 (NCBI Gene ID: 819150), WOX5 (NCBI Gene ID: 820297), and PLT4 (NCBI Gene ID: 59281483), as shown in Table 1.

**Table 1**

| *Saussurea involucrata* | NCBI genome ID of *Saussurea involucrata* | *Arabidopsis thaliana* | NCBI ID of homologous gene in *Arabidopsis thaliana* |
|---|---|---|---|
| | PRJNA991078 | WOX4(AT1G46480) | 841113 |
| SnowLotus23738 | PRJNA991078 | WOX5(AT3G11260) | 820297 |
| SnowLotus08678 | PRJNA991078 | LBD18(AT2G45420) | 819150 |
| | PRJNA991078 | PLT4(AT5G17430) | 59281483 |

The term "differentiation-related genes" refers to genes associated with the regulation of directional differentiation of the differentiated embryogenic pluripotent stem cell population.

In some embodiments, the differentiation-related genes comprise WOX4, LBD18, WOX5, and PLT4.

In some embodiments, expression levels of WOX4 and LBD18 in the differentiated embryogenic pluripotent stem cell population are less than 1/10 of expression levels of corresponding genes in the control group. For example, expression levels of WOX4 and LBD18 in the differentiated embryogenic pluripotent stem cell population may be approximately 1/11, 1/12, 1/13, 1/14, 1/15, 1/20, 1/25, 1/30, 1/50, or 1/100 of expression levels of the corresponding genes in the control group.

In some embodiments, expression levels of WOX5 and PLT4 in the differentiated embryogenic pluripotent stem cell population are less than 1/2 of expression levels of corresponding genes in the control group. For example, expression levels of WOX5 and PLT4 in the differentiated embryogenic pluripotent stem cell population may be 1/3, 1/4, 1/5, 1/6, 1/7, 1/8, 1/9, 1/10, 1/15, 1/20, 1/30, 1/40, or 1/50 of expression levels of the corresponding genes in the control group.

In some embodiments, the process of controlled differentiation may be performed based on screening of cellular characteristics. For example, the cellular characteristics may include morphology, microscopic image features, compound components, and color, or the like.

In some embodiments, through the process of controlled differentiation, the differentiated embryogenic pluripotent stem cell population may be directionally differentiated into cells, tissues, or organs favorable for the synthesis of target metabolites. For enzymes related to a metabolic pathway of the target metabolites, the cells, tissues, or organs can be favorable for maintaining expression levels of the enzymes and/or can be favorable for increasing expression levels of one or more of the enzymes. In some embodiments, the target metabolites may include at least one of one or more flavonoid compounds, one or more phenylpropanoid derivatives, and a carboxylic acid and one or more derivatives thereof, or the like.

In step b2, performing amplification culture on the differentiated embryogenic pluripotent stem cell population to harvest a culture medium containing the composition derived from the callus culture of snow lotus cells.

In some embodiments, the method further comprises performing metabolic regulation on the differentiated embryogenic pluripotent stem cell population based on flavonoid metabolic pathway enhancement during the amplification culture.

The term "flavonoid metabolic pathway enhancement" refers to an increased metabolic synthesis level of flavonoid compounds as compared to the absence of metabolic regulation.

In some embodiments, the metabolic regulation causes the differentiated embryogenic pluripotent stem cell population to possess the following features: flavonoid metabolic pathway-related genes in the differentiated embryogenic pluripotent stem cell population are expressed, and the flavonoid metabolic pathway-related genes comprise PAL (NCBI Gene ID: 818280), C4H (NCBI Gene ID: 817599), 4CL (NCBI Gene ID: 841593), CHI (NCBI Gene ID: 824678), C3H (NCBI Gene ID: 817599), F5H (NCBI Gene ID: 829779), and CAD (NCBI Gene ID: 829572), as shown in Table 2. In some embodiments, the flavonoid metabolic pathway-related genes comprise PAL, C4H, 4CL, CHI, C3H, F5H, and CAD.

**Table 2**

| *Saussurea involucrata* | NCBI genome ID of *Saussurea involucrata* | *Arabidopsis thaliana* | NCBI ID of homologous gene in *Arabidopsis thaliana* |
|---|---|---|---|
| SnowLotus16820 | PRJNA991078 | PAL(AT2G37040) | 818280 |
| SnowLotus40612 | PRJNA991078 | C4H(AT2G30490) | 817599 |
| SnowLotus13596 | PRJNA991078 | 4CL(AT1G51680) | 841593 |
| SnowLotus16695 | PRJNA991078 | CHI(AT3G55120) | 824678 |
| | PRJNA991078 | C3H(AT2G30490) | 817599 |
| | PRJNA991078 | F5H(AT4G36220) | 829779 |
| | PRJNA991078 | CAD(AT3G19450) | 829572 |

In some embodiments, the flavonoid metabolic pathway-related genes are genes encoding enzymes related to the flavonoid metabolic pathway and genes regulating expression of key enzymes in flavonoid anabolic metabolism. The metabolic regulation enables the expression of the flavonoid metabolic pathway-related genes, maintaining the expression levels of enzymes at various processes/nodes of the flavonoid metabolic pathway, thereby making a flux distribution of the metabolic pathway relatively uniform.

In some embodiments, using *in vitro* cells of the wild *Saussurea involucrata* as a control group, expressions of C3H, F5H, and CAD in the differentiated embryogenic pluripotent stem cell population are increased. The expression levels of key enzymes in the flavonoid metabolic pathway at the mRNA level were detected by quantitative real-time polymerase chain reaction (qPCR). In the embryogenic pluripotent stem cell population (purple), cinnamate 3-hydroxylase (C3H), flavonoid 3',5'-hydroxylase (F5H), and cinnamyl alcohol dehydrogenase (CAD) exhibit significantly upregulated expression, with expression levels of 1.3-fold, 2.7-fold, and 7.2-fold of the control group, respectively.

Elevated expression levels of specific flavonoid metabolic pathway-related genes can enhance the flavonoid metabolic pathway, thereby increasing the synthesis yield of metabolites.

In some embodiments, the composition derived from the callus culture of snow lotus cells in the culture medium exhibits dark purple in the viable state and grows in aggregates. The diameter of the cell aggregates of the composition derived from the callus culture of snow lotus cells ranges from 0.02 mm to 2 mm.

In some embodiments, the method further comprises separating the composition derived from the callus culture of snow lotus cells from the culture medium and preparing an extract from the composition derived from the callus culture of snow lotus cells. The extract is prepared by a process including: subjecting the composition derived from the callus culture of snow lotus cells to repeated freeze-thawing to obtain a thawed mixture; performing an extraction treatment on the thawed mixture using an aqueous ethanol solution to obtain a preliminary extract, wherein the extraction treatment includes at least one of ultrasonic extraction or stirring extraction; separating a solid part and a liquid part of the preliminary extract; and subjecting the liquid part to a drying process to obtain the extract.

In some embodiments, the number of freeze-thawing cycles is greater than or equal to 3.

In some embodiments, the freeze-thawing includes freezing the composition derived from the callus culture of snow lotus cells by cooling to -20°C or placing it in liquid nitrogen for 1.5 to 2.5 h, and subsequently thawing the composition derived from the callus culture of snow lotus cells to 4°C by water bath.

In some embodiments, the separation of the solid part and the liquid part is performed by centrifugation and/or filtration.

In some embodiments, the drying process is freeze-drying or oven-drying.

In some embodiments, the obtained extract possesses the following features: the extract is a powder with uniform texture, appears grayish purple and has uniform color; has a slight aromatic odor and a slightly sweet taste; moisture content/% ≤10, ash content/% ≤10, acid-insoluble ash content/% ≤2.0, and extractives/% ≥30.0.

The method for preparing the extract can achieve cell wall disruption of the composition derived from the callus culture of snow lotus cells through repeated freeze-thawing, which causes water in cells to freeze into ice crystals and increase in volume, while ensuring the activity of bioactive substances, thereby efficiently obtaining a high-quality extract.

Some embodiments of the present disclosure further provide a use of the aforementioned composition derived from the callus culture of snow lotus cells in the preparation of a medicament for AS.

In some embodiments, compared with a control group with AS, in a treatment group administered with the composition derived from the callus culture of snow lotus cells, the formation of atherosclerotic plaques in blood vessels is reduced.

In some embodiments, compared with a control group with AS, in a treatment group administered with the composition derived from the callus culture of snow lotus cells, the gut microbiota distribution is regulated, the regulation reduces activation and migration of macrophages, and reduces apoptosis of endothelial cells, thereby reducing the formation of atherosclerotic plaques.

In some embodiments, the medicament is used to prevent, treat, or alleviate AS.

In some embodiments, the medicament may be formulated into one of an injection, a tablet, a capsule, an oral liquid, a granule, a decoction, a powder, a pill, an ointment, a pellet, and a suppository.

Some embodiments of the present disclosure further provide a use of the composition derived from the callus culture of snow lotus cells in the preparation of a medicament for chronic long-term liver injury.

In some embodiments, compared with a model group with chronic long-term liver injury, in a treatment group administered with the composition derived from the callus culture of snow lotus cells, levels of liver function injury-related indicators are reduced, and the liver function injury-related indicators comprise alanine aminotransferase (ALT), aspartate aminotransferase (AST), gamma-glutamyltransferase (GGT), and alkaline phosphatase (ALP); and in the treatment group, generation of fibrous tissue in liver tissue is reduced, and lipid droplet formation and lipid deposition are reduced, thereby reducing the formation of alcoholic fatty liver.

In some embodiments, compared with a model group with chronic long-term liver injury, in a treatment group administered with the composition derived from the callus culture of snow lotus cells, gut microbiota distribution is regulated, and the regulation increases a quantity of beneficial bacteria and decreases a quantity of harmful bacteria, thereby reducing the difference between the gut microbiota distribution of the treatment group and the gut microbiota distribution of the normal group.

In some embodiments, the medicament is used to prevent, treat, or alleviate chronic long-term liver injury.

In some embodiments, the medicament may be formulated as one of an injection, a tablet, a capsule, an oral liquid, a granule, a decoction, a powder, a pill, an ointment, a pellet, and a suppository.

The experimental methods in the following examples, unless otherwise specified, are conventional methods. The experimental materials used in the following examples, unless otherwise specified, are purchased from conventional biochemical reagent companies. The quantitative experiments in the following examples are each performed in three replicates, and the results are averaged.

### EXAMPLE 1: CONTROLLED DIFFERENTIATION AND METABOLIC REGULATION OF CELLS

Plant stem cells exist in a special microenvironment and are co-regulated by complex endogenous signals and exogenous signals. A positive and negative feedback loop formed by differentiation-related genes is one of the key mechanisms for maintaining the stability of stem cells.

A differentiated embryogenic pluripotent stem cell population (purple) suitable for the composition derived from the callus culture of snow lotus cells was obtained by a process of controlled differentiation. The expression levels of the differentiation-related genes were determined by using qPCR, and the differentiation-related genes comprise WOX4, LBD18, WOX5, and PLT4. WOX4 is a key gene related to cell division in the cambium and is a hub for regulating cambium activity. WOX4 can respond to a plurality of hormones including auxin and small peptide signals, thereby participating in maintaining cambium activity. The transcription factor LBD18 is a key factor for controlling callus formation in plant in vitro regeneration. LBD18 controls callus formation by ectopic activation of lateral root initiation signals, thereby mimicking an auxin-induced callus initiation process. WOX5 plays an important role in fate determination of a stem cell quiescent center (QC). PLT4, also referred to as BABY BOOM (BBM), is a plant-specific transcription factor of the APETALA2/ethylene-responsive factor (AP2/ERF) family, which plays an important regulatory role during the somatic embryogenesis process. As a well-known factor, it is widely used to improve the genetic transformation efficiency of transgenes. The expression levels of the differentiation-related genes were quantitatively determined by Quant StudioTM 6 Flex (ABI), and primer information is shown in Table 3.

**Table 3 Polymerase chain reaction (PCR) primer sequence information for RT-qPCR**

| Gene | Primer | Sequence (5' to 3') |
|---|---|---|
| WOX4 | Forward | GGGACAAGGTGGAACCCGAC (SEQ ID NO: 1) |
| | Reverse | CTGTCTCTCGCGGGCTTTGT (SEQ ID NO: 2) |
| WOX5 | Forward | TGGCAAGATCGAGAGCAAGA (SEQ ID NO: 3) |
| | Reverse | AAAGAGCTGCAACGTCTCGA (SEQ ID NO: 4) |
| LBD18 | Forward | ACGCCACAATTAGCAAGCCT (SEQ ID NO: 5) |
| | Reverse | GATGAGGCGGTCGTAAGGTC (SEQ ID NO: 6) |
| PLT4 | Forward | GCCGTAGATGTTGCCGGAGG (SEQ ID NO: 7) |
| | Reverse | TTCCTGCGCAGAGAGGCAAC (SEQ ID NO: 8) |

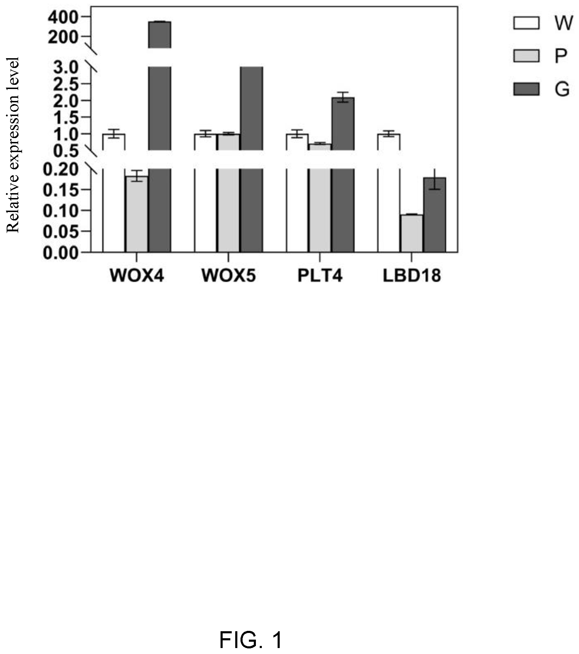

FIG. 1 is an interval bar chart illustrating relative expression levels of differentiation-related genes in an embryogenic pluripotent stem cell population at an mRNA level according to some embodiments of the present disclosure. An experimental group (P) is a sample of the aforementioned embryogenic pluripotent stem cell population, a control group (W) is sample 1 of *in vitro* cells of *Saussurea involucrata* without a process of controlled differentiation, and (G) is sample 2 of *in vitro* cells of *Saussurea involucrata* without a process of controlled differentiation. As shown in FIG. 1, compared with group W, the expression of WOX4 and LBD18 in group P is downregulated by 5.56-fold and 11.04-fold, respectively, and the relative expression levels of WOX5 and PLT4 in group P basically remain at the same level.

For the metabolically regulated embryogenic pluripotent stem cell population, the expression levels of its secondary metabolic key enzymes were detected using qPCR. The secondary metabolic key enzymes include phenylalanine ammonia-lyase 1 (gene name: PAL1), chalcone isomerase (gene name: CHI), flavonoid-3'-hydroxylase-1 (gene name: F3H-1), flavonoid-3'-hydroxylase-2 (gene name: F3H-2), dihydroflavonol 4-reductase (gene name: DFR1), anthocyanidin synthase (gene name: ANS), cinnamate-4-hydroxylase (gene name: C4H), 4-coumarate coenzyme A ligase (gene name: 4CL), cinnamate-3-hydroxylase (gene name: C3H), ferulate 5-hydroxylase (F5H) (gene name: F5H), cinnamoyl-CoA reductase (gene name: CCR), cinnamyl alcohol dehydrogenase (gene name: CAD), uridine diphosphate glycosyltransferase (gene name: UGT), and chalcone synthase (gene name: CHS). The expression levels of flavonoid metabolic key enzyme-related genes are quantitatively determined by Quant StudioTM 6 Flex (ABI). Primer information is shown in Table 4.

**Table 4 PCR primer sequence information for RT-qPCR**

| Gene | Primer | Sequence (5' to 3') |
|---|---|---|
| PAL1 | Forward | GGTGAAGGCGAGTAGTGATTGG (SEQ ID NO: 9) |
| | Reverse | GCTGAATGTGGAAGGGTGTGA (SEQ ID NO: 10) |
| C4H | Forward | TGACACCAAACTCGGACCAG (SEQ ID NO: 11) |
| | Reverse | ACCAGGCGTTGACCAAGAT (SEQ ID NO: 12) |
| 4CL | Forward | GACGAGTGTAGCACAGCAAGT (SEQ ID NO: 13) |
| | Reverse | ACGCACATCAGCACATCCT (SEQ ID NO: 14) |
| C3H | Forward | GCCGCCATCGTCTTCATTC (SEQ ID NO: 15) |
| | Reverse | CGTAGGTTCTCGCCCATTCA (SEQ ID NO: 16) |
| F5H | Forward | GTCAACGCATACGCCATCAAC (SEQ ID NO: 17) |
| | Reverse | CGCCATTTCCATCGCATACAAC (SEQ ID NO: 18) |
| CCR | Forward | GACGAGGCTAAGGCGAGAG (SEQ ID NO: 19) |
| | Reverse | TGGACCGAATTGGCGTAGG (SEQ ID NO: 20) |
| CAD | Forward | CCATTGCTTAGTGTGCTCAAACC (SEQ ID NO: 21) |
| | Reverse | AGGGAGTTGGCGACATCAATC (SEQ ID NO: 22) |
| CHS | Forward | GACACGCTTCGGCTACAGAT (SEQ ID NO: 23) |
| | Reverse | AATGGACGCACTATGGAGGAC (SEQ ID NO: 24) |
| CHI | Forward | CATTCCGTCACTCGCCGTTA (SEQ ID NO: 25) |
| | Reverse | TGTAGGTCCCTTGTGCTTTCC (SEQ ID NO: 26) |
| F3H-1 | Forward | ACTACCCGAAATGCCCTCAG (SEQ ID NO: 27) |
| | Reverse | TCTTGCCACCATCACGAGTC (SEQ ID NO: 28) |
| F3H-2 | Forward | GGATGACGCCGATGGAGAA (SEQ ID NO: 29) |
| | Reverse | CGATGGGTGGAGCCTGAAG (SEQ ID NO: 30) |
| DFR1 | Forward | TCCATTCATCACTCCTTCGTTCC (SEQ ID NO: 31) |
| | Reverse | TCTTCCTTTGGCTTTAGGGTTCTC (SEQ ID NO: 32) |
| ANS | Forward | ACAACGCCCGACGATTACAT (SEQ ID NO: 33) |
| | Reverse | CCCGAGAGATAGAACCGAGAGT (SEQ ID NO: 34) |
| UGT | Forward | TGGTTCCACTCCCACCCTAA (SEQ ID NO: 35) |
| | Reverse | TCAAGAACGGCGGTCAAGAA (SEQ ID NO: 36) |
| ELF5A | Forward | ATGTCGGACGAAGAGCATCA (SEQ ID NO: 37) |
| | Reverse | ACGATGTGACCTCCTTTGCG (SEQ ID NO: 38) |

| | | |
|---|---|---|
| Note: ELF5A is a reference gene. | | |

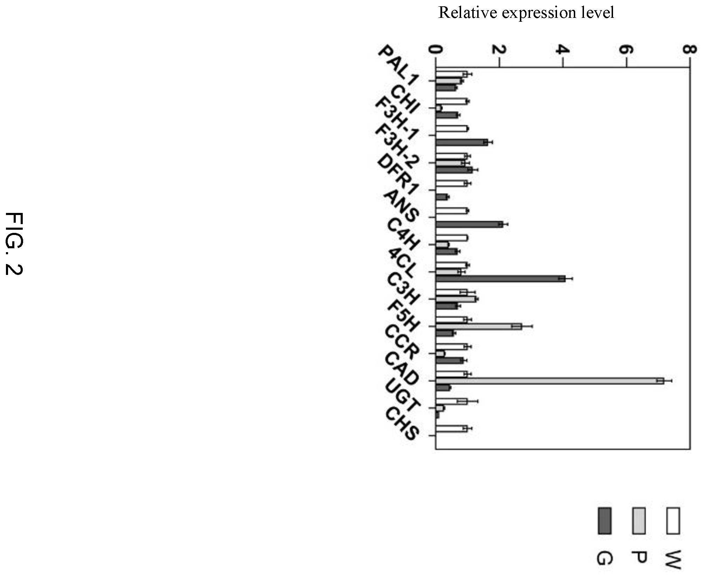

FIG. 2 is an interval bar chart illustrating the relative expression levels of the key enzymes in the flavonoid metabolic pathway at the mRNA level according to some embodiments of the present disclosure. The experimental group (P) is a sample of the aforementioned metabolically regulated embryogenic pluripotent stem cell population, the control group (W) is sample 1 of the *in vitro* cells of the *Saussurea involucrata* without the process of controlled differentiation, and (G) is sample 2 of the *in vitro* cells of the *Saussurea involucrata* without the process of controlled differentiation. As shown in FIG. 2, compared with the group P, the group W exhibits significant upregulation in the expressions of C3H, F5H, and CAD, which are 1.3-fold, 2.7-fold, and 7.2-fold that of the control group, respectively. The high expressions of C3H, F5H, and CAD cause the metabolism of phenolic compounds (e.g., small-molecule phenolic acids, lignans), flavonoid compounds, and isoflavonoid compounds in the composition derived from the callus culture of snow lotus cells via the phenylpropanoid (PAL) pathway to be higher than that of ordinary wild plants.

### EXAMPLE 2: DETECTION OF HPLC CHARACTERISTIC CHROMATOGRAM OF COMPOSITION DERIVED FROM THE CALLUS CULTURE OF SNOW LOTUS CELLS

### Establishment of the HPLC characteristic chromatogram of the composition derived from the callus culture of snow lotus cells

Chromatographic conditions: using octadecylsilane-bonded silica gel as a packing material; using methanol as mobile phase A; using a 0.3% aqueous formic acid solution as mobile phase B; a flow rate of 0.9 mL/min and a detection wavelength of 265 nm; and the gradient elution conditions are as follows:
From 0.01 to 15 min, mobile phase A: 20%, mobile phase B: 80%;
From 15 to 25 min, mobile phase A: 20% to 36%, mobile phase B: 80% to 64%;
From 25 to 60 min, mobile phase A: 36% to 37%, mobile phase B: 64% to 63%;
From 60 to 85 min, mobile phase A: 37% to 70%, mobile phase B: 63% to 30%;
From 85 to 90 min, mobile phase A: 70% to 100%, mobile phase B: 30% to 0%;
From 90 to 100 min, mobile phase A: 100%, mobile phase B: 0%;
From 100 to 100.01 min, mobile phase A: 100% to 20%, mobile phase B: 0% to 80%;
At 115 min, stop.

Preparation of the reference solution: accurately weigh appropriate amounts of a syringin reference substance, a chlorogenic acid reference substance, and a 1,5-dicaffeoylquinic acid reference substance, and add 60% methanol to prepare a solution containing 0.05 mg/mL of syringin, 0.08 mg/mL of chlorogenic acid, and 0.3 mg/mL of the 1,5-dicaffeoylquinic acid. Take an appropriate amount of a rutin reference substance and add 60% methanol to prepare a reference solution for positioning.

Preparation of the test solution: accurately weigh about 0.2 g of the powder of this product (passed through a No. 4 sieve) (in this example, the reference medicinal material of *Saussurea involucrata* was purchased from the National Institutes for Food and Drug Control, with a sample weight of 0.5 g), and place it in a stoppered conical flask, accurately add 25 mL of 60% methanol, weigh the total mass, perform ultrasonic treatment for 10 min (a power of 250 W, a frequency of 40 kHz), then remove and cool to room temperature, replenish the lost weight with 60% methanol, shake well, and filter through a 0.45 µm microporous membrane to obtain the test solution.

Measurement: prepare a plurality of batches of composition samples derived from the callus culture of snow lotus cells into test solutions, accurately pipette 5 µL of the reference solution and 5 µL of each of the test solutions, inject them into a liquid chromatograph (a Shimadzu LC-2030C 3D high-performance liquid chromatograph with a Phenomenex Gemini 5 µm C18 250×4.6 chromatographic column), measure and record the chromatograms.

Identification of characteristic peaks: according to the principles that the relative retention time is stable, and the peaks could be detected in all batches of samples with relatively high peak intensities, a total of 9 peaks with good reproducibility were selected as characteristic peaks. The results show that when peak 5 is used as a reference peak (peak S), the relative standard deviations (RSD) of the relative retention time of the other characteristic peaks are within an acceptable range.

### Identification of the characteristic peaks in the HPLC characteristic chromatogram

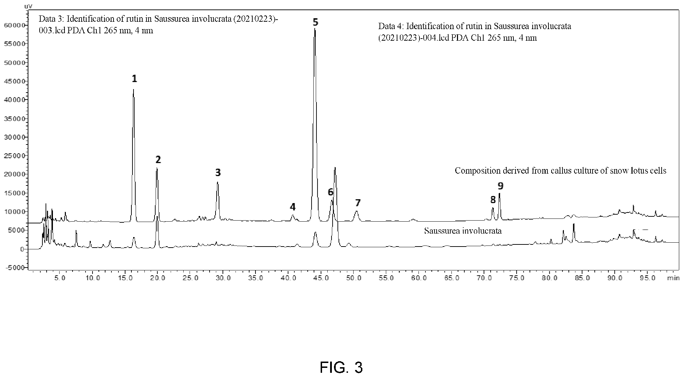

FIG. 3 is a chromatographic comparison chart of characteristic peaks of a composition derived from a callus culture of snow lotus cells and *Saussurea involucrata* at 265 nm according to some embodiments of the present disclosure. By comparing the HPLC chromatograms of the composition derived from the callus culture of snow lotus cells and the plant-derived *Saussurea involucrata*, it can be found that the composition derived from the callus culture of snow lotus cells contains more components in higher amounts than the *Saussurea involucrata*; among the 9 characteristic peaks of the composition derived from the callus culture of snow lotus cells, peak 1, peak 2, peak 4, and peak 5 are common to the composition derived from the callus culture of snow lotus cells and the plant-derived *Saussurea involucrata*; and compared with the composition derived from the callus culture of snow lotus cells, a peak at a position similar to peak 6 in the plant-derived *Saussurea involucrata* shows a shift in RT, and the RT delays by 0.5 min.

Using ultraviolet spectroscopy to determine the ultraviolet spectra of the compounds corresponding to the characteristic peaks of the composition derived from the callus culture of snow lotus cells, and by comparing with the ultraviolet spectra of the reference substances, the following identifications can be made.

The compound corresponding to peak 1 of the composition derived from the callus culture of snow lotus cells is syringin.

The compound corresponding to peak 2 of the composition derived from the callus culture of snow lotus cells is chlorogenic acid.

The compound corresponding to peak 4 of the composition derived from the callus culture of snow lotus cells is 1,4-dicaffeoylquinic acid.

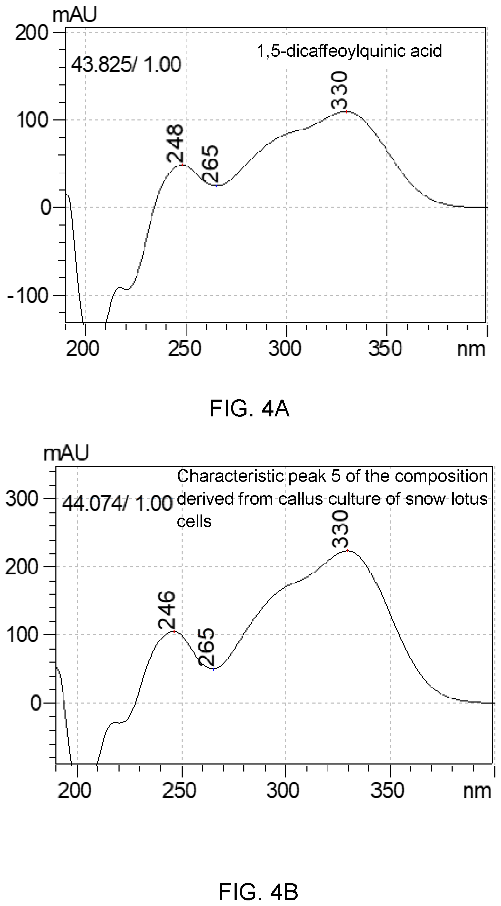

The compound corresponding to peak 5 of the composition derived from the callus culture of snow lotus cells is 1,5-dicaffeoylquinic acid (FIGs. 4A and 4B).

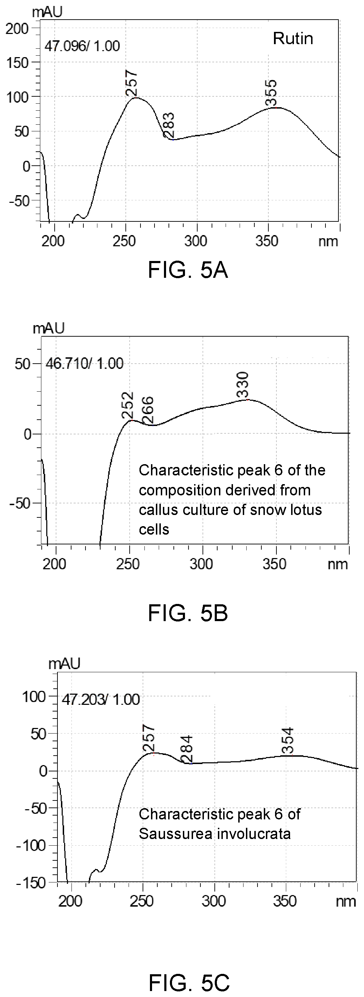

The maximum ultraviolet absorption wavelengths (252 nm, 330 nm) of peak 6 of the composition derived from the callus culture of snow lotus cells is inconsistent with those of the rutin reference substance (FIGs. 5A and 5B), the compound corresponding to peak 6 of the composition derived from the callus culture of snow lotus cells is not rutin, while the peak at a position similar to peak 6 in *Saussurea involucrata* shows the maximum ultraviolet absorption wavelengths consistent with those of the rutin reference substance (FIGs. 5A and 5C), indicating that the peak at the position similar to peak 6 in *Saussurea involucrata* is rutin. By comparing with the ultraviolet spectrum of the 3,5-dicaffeoylquinic acid reference substance, it can be known that the compound corresponding to peak 6 is 3,5-dicaffeoylquinic acid.

The compound corresponding to peak 7 of the composition derived from the callus culture of snow lotus cells is dicaffeoylquinic acid malate ester.

The compound corresponding to peak 8 of the composition derived from the callus culture of snow lotus cells is 1,3,5-tricaffeoylquinic acid malate ester.

The compound corresponding to peak 9 of the composition derived from the callus culture of snow lotus cells is 1,3,5-tricaffeoylquinic acid methyl malate ester.

Therefore, *Saussurea involucrata* contains syringin, chlorogenic acid, and 1,5-dicaffeoylquinic acid, and also contains rutin; while the composition derived from the callus culture of snow lotus cells contains more components in higher amounts than *Saussurea involucrata.*

### EXAMPLE 3: DETERMINATION OF COMPONENT AMOUNTS OF THE COMPOSITION DERIVED FROM THE CALLUS CULTURE OF SNOW LOTUS CELLS

The amounts of various components in a plurality of batches of the composition samples derived from the callus culture of snow lotus cells were determined by using the above-mentioned HPLC method. The results are shown in Table 5.

**Table 5**

| Component | Amount (mg/g culture) |
|---|---|
| Peak 1 (syringin) | 31±19 |
| Peak 2 (chlorogenic acid) | 8±5 |
| Peak 3 (unknown) | |
| Peak 4 (1,4-dicaffeoylquinic acid) | 7±3 |
| Peak 5 (1,5-dicaffeoylquinic acid) | 46±31 |
| Peak 6 (3,5-dicaffeoylquinic acid) | 13±7 |
| Peak 7 (dicaffeoylquinic acid malate ester) | 11±7 |
| Peak 8 (1,3,5-tricaffeoylquinic acid malate ester) | 9±7 |
| Peak 9 (1,3,5-tricaffeoylquinic acid methyl malate ester) | 13±8 |

### EXAMPLE 4: EVALUATION OF ANTIOXIDANT ACTIVITY OF THE COMPOSITION DERIVED FROM THE CALLUS CULTURE OF SNOW LOTUS CELLS

### Biological materials

HUVECs: HUVECs obtained from the ATCC cell bank were cultured in endothelial cell medium (ECM) (Sciencell), supplemented with 10% fetal bovine serum (Sangon Biotech) and 1% penicillin/streptomycin solution (Gibco, Thermo Fisher). The HUVECs were maintained at 37°C in a humidified atmosphere containing 5% CO₂.

### Methods

In this example, the HUVECs and H₂O₂ were used to simulate initial injury to endothelial function during the development of AS, and the antioxidant activity of the composition derived from the callus culture of snow lotus cells was evaluated by analyzing its ameliorative effect on the foregoing injury.

### Experimental group design

Control group (Con): treated with a complete ECM.

Model group (H₂O₂): subjected to oxidative stress induction with 650 µM H₂O₂ for 4 h.

Protection group (SI): pre-treated with the composition derived from the callus culture of snow lotus cells (10 µg/mL) for 4 h, and then subjected to oxidative stress induction with 650 µM H₂O₂ for 4 h.

### Cell viability assay (CCK-8)

The HUVECs were seeded into 96-well plates at a density of 10,000 cells per well, and cell viability was evaluated according to the experimental group design described in this example. Briefly, after 4 h of H₂O₂ treatment, a CCK8 reagent (DOJINDO LABORATORIES) was added to the wells of each group at a volume ratio of 1:100 for 2 h of treatment. Then, the absorbance was measured at 450 nm using a Microplate Reader (SpectraMax i3X).

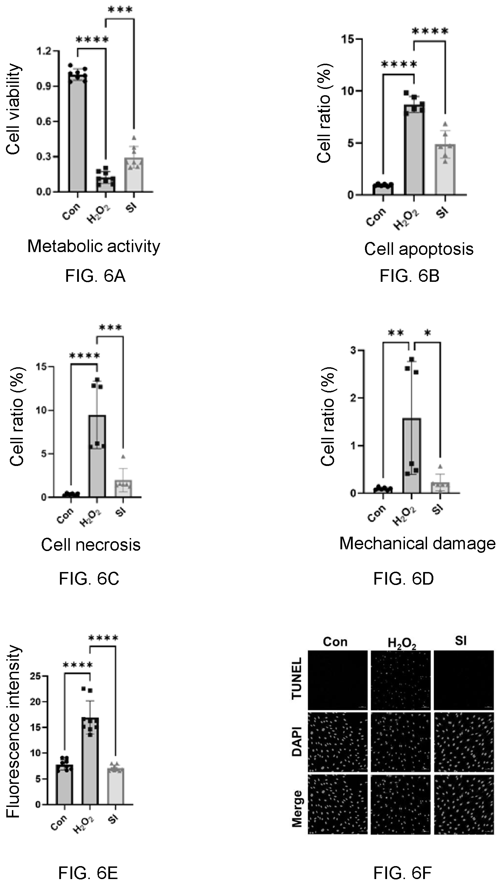

FIG. 6A is an interval bar chart illustrating cell viability of HUVECs in different groups according to some embodiments of the present disclosure. As shown in FIG. 6A, compared with the HUVECs subjected to oxidative stress in the H₂O₂ group, the cell viability of the HUVECs in the SI group, as reflected by metabolic activity, is improved due to the addition of the composition derived from the callus culture of snow lotus cells. Apoptosis detection-Annexin V-allophycocyanin (APC)/propidium iodide (PI) double staining

The HUVECs were seeded into 6-well plates at a density of 200,000 cells per well and incubated for 48 h. Then, according to the experimental group design described in this example, apoptosis was detected by flow cytometry. Briefly, after 4 h of H₂O₂ treatment, the cells were digested with trypsin, collected, and washed 2-3 times with phosphate-buffered saline (PBS). The cells were resuspended in 100 µL of a binding buffer, and 5 µL of Annexin V-APC and 5 µL of Propidium Iodide (PI) were added to the cell suspension for staining. The cell suspension was incubated at room temperature in the dark for 30 min. The incubated cell suspension was diluted to 500 µL with the binding buffer, and then fluorescence signals were detected using a flow cytometer (BD FACSCelesta). Flowjo software was used to analyze the detection data.

FIGs. 6B-6D are interval bar charts illustrating the cell apoptosis according to some embodiments of the present disclosure. The interval bar charts were determined by flow cytometry based on Annexin V-APC/PI double-stained HUVECs in different groups. As shown in FIGs. 6B-6D, compared with the H₂O₂ group, the addition of the composition derived from the callus culture of snow lotus cells significantly reduces H₂O₂-induced cell apoptosis, necrosis, and mechanical damage in the SI group.

### Apoptosis detection-TUNEL assay

The HUVECs were seeded into eight-well plates (µ-Slide 8 Well high, ibidi) at a density of 20,000 cells per well and incubated for 24 h. Then, according to the experimental group design described in this example, apoptosis was detected using the TUNEL assay. Briefly, after 4 h of H₂O₂ treatment, the cells of each group were stained using a TUNEL BrightRed Apoptosis Detection Kit (Vazyme), and fluorescence signals were observed at a wavelength of 620 nm using a laser confocal microscope (ZEISS LSM 980). In addition, fluorescence imaging was also performed on the DAPI-stained nuclei in each group using a laser confocal microscope, and merged fluorescence images of TUNEL and DAPI double staining were obtained through an image Merge technique.

FIG. 6E is an interval bar chart illustrating the fluorescence intensity according to some embodiments of the present disclosure. The interval bar chart was determined by flow cytometry based on TUNEL-stained HUVECs in different groups. FIG. 6F is a fluorescence image according to some embodiments of the present disclosure. The fluorescence image was obtained by the laser confocal microscope based on TUNEL/DAPI double-stained HUVECs in different groups. As shown in FIGs. 6E and 6F, compared with the H₂O₂ group, the addition of the composition derived from the callus culture of snow lotus cells significantly reduces H₂O₂-induced cell apoptosis, necrosis, and mechanical damage in the SI group.

### Intracellular ROS detection-ROS fluorescence probe assay

The HUVECs were seeded into 6-well plates at a density of 200,000 cells per well. When the cell density reached 80%, the production of intracellular ROS was detected using the ROS fluorescence probe assay according to the experimental group design described in this example. Briefly, after 4 h of H₂O₂ treatment, the cells were rinsed 2-3 times with a serum-free medium. The cells were stained with a ROS-specific probe 2',7'-dichlorodihydrofluorescein diacetate (DCFH-DA) (GlpBio) at a concentration of 1:1000 and incubated at 37°C in the dark for 30 min. Subsequently, green fluorescence at 525 nm was detected using flow cytometry after cell collection. In addition, fluorescence imaging was also performed on the DAPI-stained nuclei in each group using the laser confocal microscope, and merged fluorescence images of DCFH-DA/DAPI double staining were obtained through an image Merge technique.

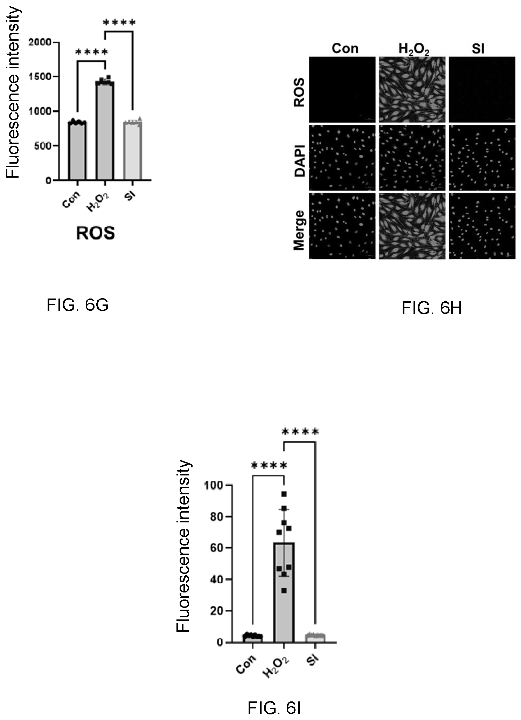

FIG. 6G is an interval bar chart illustrating the fluorescence intensity from quantitative analysis of ROS production by HUVECs in different groups using flow cytometry according to some embodiments of the present disclosure. FIG. 6H is a fluorescence image according to some embodiments of the present disclosure. The fluorescence image was obtained by the laser confocal microscope based on DCFH-DA/DAPI double-stained HUVECs in different groups. FIG. 6I is an interval bar chart illustrating the fluorescence intensity of HUVECs in different groups based on DCFH-DA staining according to some embodiments of the present disclosure. As shown in FIGs. 6G-6I, compared with the H₂O₂ group, the intracellular ROS level of the SI group is significantly reduced, which is similar to the intracellular ROS level of the Con group. The composition derived from the callus culture of snow lotus cells exhibits a strong ability to inhibit the production and release of intracellular ROS in the HUVECs.

In summary, under the H₂O₂-induced oxidative stress, the composition derived from the callus culture of snow lotus cells exhibits good antioxidant activity.

### EXAMPLE 5: EVALUATION OF ANTI-INFLAMMATORY ACTIVITY OF THE COMPOSITION DERIVED FROM THE CALLUS CULTURE OF SNOW LOTUS CELLS

### Biological materials

Macrophages: RAW264.7 cells were transferred to a 25 cm² cell culture flask and grown in a Dulbecco's Modified Eagle Medium (DMEM) containing 10% fetal bovine serum (FBS) and a 1% penicillin-streptomycin solution (100 U/mL penicillin, 100 µg/mL streptomycin) in a 5% CO₂ atmosphere at 37°C.

Oxidized low-density lipoprotein (ox-LDL): ox-LDL (1 mg/mL) was incubated with 10 µmol/L CuSO₄ at 37°C for 18 h to induce deep oxidation, thereby obtaining the oxidized ox-LDL.

### Methods

### Effect of the composition derived from the callus culture of snow lotus cells on expression levels of pro-inflammatory and anti-inflammatory factors

In this example, lipopolysaccharide (LPS, Sigma) was used as an inflammatory stimulant to activate a mouse macrophage RAW264.7 cell line to construct an inflammatory response cell model. The inflammatory response cell model was used to evaluate the anti-inflammatory activity of the composition derived from the callus culture of snow lotus cells.

RAW264.7 cells were seeded into 6-well plates at a density of 1×10⁵ cells/well and incubated for 24 h. Then, anti-inflammatory activity was evaluated according to the following experimental group design.

Control group (Con): RAW264.7 cells were cultured normally without LPS stimulation.

Model group (LPS): RAW264.7 cells were stimulated with LPS (0.5 µg/mL) for 24 h.

Protection group (SI): RAW264.7 cells were pre-treated with the composition derived from the callus culture of snow lotus cells at different concentrations (1 µg/mL, 10 µg/mL) for 4 h, and then stimulated with LPS (0.5 µg/mL) for 24 h.

After the LPS treatment, total Ribonucleic acid (RNA) was extracted from each group of the samples using a TRIzol reagent (Sigma), and the quantity and purity of the RNA were measured using a NanoDrop 1000 spectrophotometer (Thermo Fisher Scientific). The Abs 260/280 nm ratio of all the samples was within a range of 1.8 to 2.0. Then, complementary deoxyribonucleic acid (cDNA) was synthesized from the extracted total RNA using an Evo M-MLV RT Mix Kit with gDNA Clean for qPCR Ver.2 AG11728 (AG). Reverse Transcription-quantitative Polymerase Chain Reaction (RT-qPCR) analysis was performed using a SYBR Green Premix Pro Taq HS qPCR Kit AG11701 (AG), and the primer information is shown in Table 6. The expression levels of pro-inflammatory factors Tumor Necrosis Factor-alpha (TNF-α), Interleukin-6 (IL-6), and Interleukin-1 beta (IL-1β), and anti-inflammatory factor Interleukin-10 (IL-10) were quantitatively determined by a QuantStudio^{™} 6 Flex (ABI).

**Table 6 PCR primer sequence information for RT-qPCR**

| Gene | Primer | Sequence |
|---|---|---|
| TNF-α | Forward | GCCTCTTCTCATTCCTGCTTGTGG (SEQ ID NO: 39) |
| | Reverse | GTGGTTTGTGAGTGTGAGGGTCTG (SEQ ID NO: 40) |
| IL-6 | Forward | CTTCTTGGGACTGATGCTGGTGAC (SEQ ID NO: 41) |
| | Reverse | AGGTCTGTTGGGAGTGGTATCCTC (SEQ ID NO: 42) |
| IL-1β | Forward | TCGCAGCAGCACATCAACAAGAG (SEQ ID NO: 43) |
| | Reverse | AGGTCCACGGGAAAGACACAGG (SEQ ID NO: 44) |
| IL-10 | Forward | TCCCTGGGTGAGAAGCTGAAGAC (SEQ ID NO: 45) |
| | Reverse | CACCTGCTCCACTGCCTTGC (SEQ ID NO: 46) |
| GAPDH | Forward | ACGGCAAATTCAACGGCACAG (SEQ ID NO: 47) |
| | Reverse | ACACCAGTAGACTCCACGACATAC (SEQ ID NO: 48) |

| | | |
|---|---|---|
| Note: Glyceraldehyde-3-phosphate dehydrogenase (GAPDH) is a reference gene. | | |

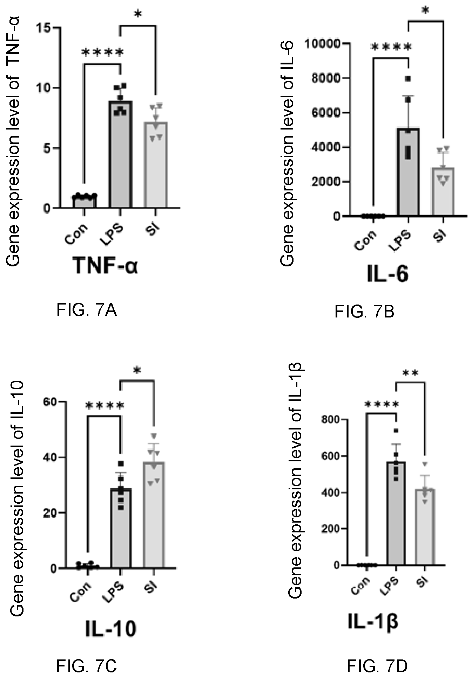

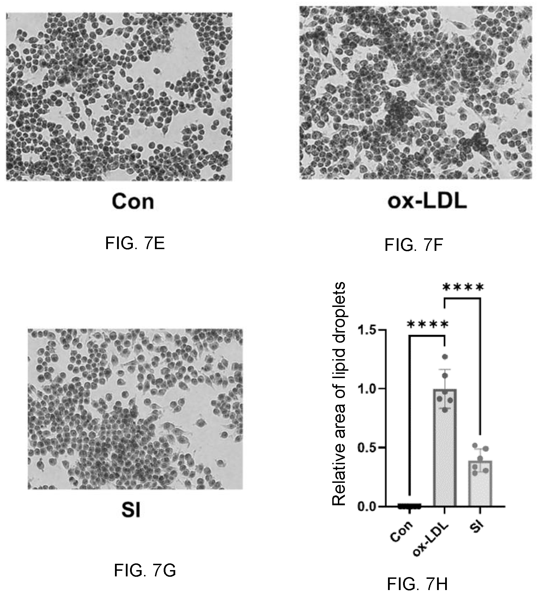

FIGs. 7A-7D are bar statistical graphs illustrating gene expression levels of pro-inflammatory factors and anti-inflammatory factors in RAW264.7 cells from different groups according to some embodiments of the present disclosure. As shown in FIGs. 7A-7D, compared with the LPS group, the gene expression of pro-inflammatory factors TNF-α, IL-6, and IL-1β in the SI group is downregulated, while the gene expression of anti-inflammatory factor IL-10 is upregulated. This indicates that the composition derived from the callus culture of snow lotus cells exhibits a certain degree of anti-inflammatory activity.

### Effect of the composition derived from the callus culture of snow lotus cells on foam cell formation

Foam cells are important contributors to the development of atherosclerotic plaques, and foam cell formation is induced by macrophages engulfing ox-LDL. In this example, RAW264.7 cells and ox-LDL were used to simulate foam cell formation during the development of AS, and the effect of the composition derived from the callus culture of snow lotus cells on foam cell formation was evaluated. Briefly, the evaluation was performed according to the following experimental group design.

Normal group (Con): RAW264.7 cells were normally cultured, and the medium was replaced every two days.

Model group (ox-LDL): RAW264.7 cells were cultured with oxidized ox-LDL, and the medium was replaced every two days.

Treatment group (SI): RAW264.7 cells were cultured with oxidized ox-LDL, and the composition derived from the callus culture of snow lotus cells (0.01 µg/mL), and the medium was replaced every two days.

After 14 days of culture, the cells from each group were subjected to Oil Red O staining and observed under a microscope.

FIGs. 7E-7G are images of Oil Red O-stained RAW264.7 cells in different groups according to some embodiments of the present disclosure. FIG. 7H is a bar chart illustrating a relative area of lipid droplets in RAW264.7 cells in different groups according to some embodiments of the present disclosure. As shown in FIGs. 7E-7G, compared with the Con group, oil red O-positive cells appear in the images of the ox-LDL group, which indicates that the addition of ox-LDL promotes foam cell formation in RAW264.7 cells. In contrast, compared with the ox-LDL group, the number of positively stained cells in the SI group with the addition of the composition derived from the callus culture of snow lotus cells is reduced, which indicates that the composition derived from the callus culture of snow lotus cells has a potential capacity in inhibiting foam cell formation. The quantitative analysis result shown in FIG. 7H further confirms that the composition derived from the callus culture of snow lotus cells has a significant inhibitory effect on foam cell formation.

In summary, in the inflammatory cell model and the foam cell formation model simulating the development of AS, the composition derived from the callus culture of snow lotus cells exhibits good anti-inflammatory activity.

### EXAMPLE 6: EFFECT OF THE COMPOSITION DERIVED FROM THE CALLUS CULTURE OF SNOW LOTUS CELLS ON ATHEROSCLEROTIC PLAQUE FORMATION

### Biological materials

AS model mice (ApoE) and C57BL/6J mice: 8 weeks old, male, ear-tagged, with a body weight ranging from 18 g to 22 g. The mice were housed at the Animal Experiment Center of Zhengzhou University, and specific feeding conditions included a 12-hour light/12-hour dark cycle, a normal diet, and ad libitum access to purified water.

### Methods

In this example, high-fat diet-fed ApoE mice were employed to evaluate the therapeutic effect of the composition derived from the callus culture of snow lotus cells on AS.

### Experimental Group Design

Blank Control Group (Bla): age- and sex-matched wild-type C57BL/6J mice (n=5) were normally fed (flour 5%, oatmeal 25%, cornmeal 35%, soybean flour 19%, fish meal 8%, sesame 1%, bone meal 4%, vitamin and yeast powder 2%, and refined salt 1%).

Control Group (Con): age- and sex-matched wild-type C57BL/6J mice (n=4) were high-fat fed (regular feed 49.9%, protein powder 10%, lard 22%, cholesterol 2%, bile salt 0.1%, and sugar 16%).

Model Group (Dis): ApoE mice (n=4) were fed a high-fat diet and administered by gavage with sodium carboxymethyl cellulose at a dose of 500 mg/kg, once a day for 16 weeks.

Experimental Group (SI): ApoE mice (n=5) were fed a high-fat diet and administered by gavage with the composition derived from the callus culture of snow lotus cells at a dose of 500 mg/kg, once a day for 16 weeks.

### Blood sample analysis

On day 1 and day 7, blood samples were collected from the mice of each group. The concentrations of key factors in the blood samples, including glucose (GLU), cholesterol (CHOL), triglyceride (TG), high-density lipoprotein (HDL), and low-density lipoprotein (LDL), were measured.

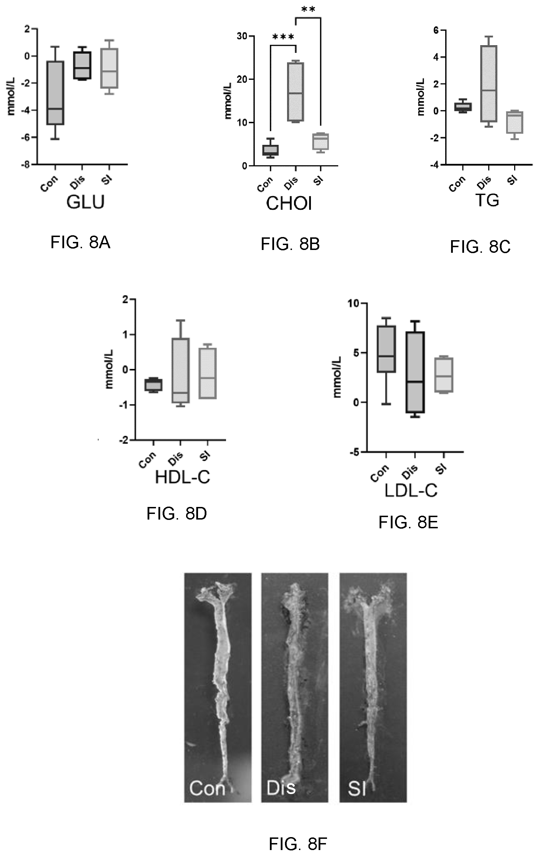

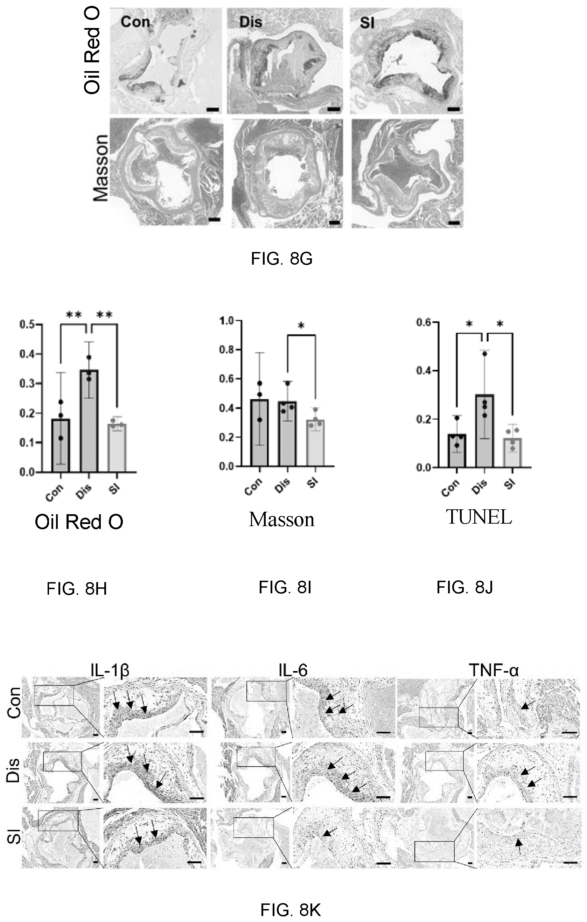

FIGs. 8A-8E are interval bar charts illustrating the concentrations of key factors in the blood of mice in different groups according to some embodiments of the present disclosure. As shown in FIGs. 8A-8E, compared with the Dis group, the growth rate of the CHOL concentration in the blood of mice of the SI group is significantly slowed between the two time points, which indicates that the composition derived from the callus culture of snow lotus cells has therapeutic potential for certain diseases, such as lipid disorders, hyperlipidemia, and/or hypercholesterolemia.

### Tissue/cell staining analysis

At the experimental endpoint, the arteries and thoracic aortas of the mice of each group were collected for tissue/cell staining and analysis: (1) observing and analyzing the formation of plaques on the thoracic aorta by Oil Red O staining; (2) identifying fibrin-positive regions in the plaques on the thoracic aorta by Masson's trichrome staining; (3) detecting and analyzing cell apoptosis by TUNEL staining; and (4) observing and analyzing inflammatory responses around the arterial wall by IHC staining for IL-1β, IL-6, and TNF-α.

FIG. 8F is an Oil Red O staining image of the thoracic aortas of mice in different groups according to some embodiments of the present disclosure. As shown in FIG. 8F, compared with the Dis group, the accumulation of plaques on the arterial wall of mice in the SI group is reduced.

FIG. 8G is a staining image of tissue sections of the thoracic aortas of mice in different groups according to some embodiments of the present disclosure. As shown in FIG. 8G, although fibrous tissue is observed in the Masson-stained images of the thoracic aortas of mice in the three groups, the SI group has a smaller area of fibrous tissue.

FIGs. 8H-8J are bar charts of quantitative analysis of Oil Red O staining, Masson's trichrome staining, and TUNEL staining according to some embodiments of the present disclosure, which demonstrate that the composition derived from the callus culture of snow lotus cells has a statistically significant effect in reducing plaque formation, reducing fibrous tissue area, and reducing cell apoptosis.

FIG. 8K is an IHC staining image of tissue sections of arteries of mice in different groups according to some embodiments of the present disclosure. As shown in FIG. 8K, compared with the Dis group, local inflammation surrounding the arterial wall is inhibited in the SI group, suggesting that the composition derived from the callus culture of snow lotus cells possesses moderate anti-inflammatory activity.

### Gut microbiota diversity analysis

Sample collection: At the experimental endpoint, fecal samples were collected from the mice of each group and stored at -80°C for further analysis.

DNA extraction, amplification, and sequencing: Total genomic DNA was extracted from the fecal samples. The quantity and quality of the DNA were measured using a spectrophotometer and agarose gel electrophoresis. PCR amplification was performed on the V3-V4 region of the bacterial 16S rRNA gene using specific primers, the primers including sample-specific 7-base barcodes for multiplex sequencing. The paired-end sequencing (2×250 bp) was performed using the Illumina NovaSeq platform.

### Sequence and bioinformatic analysis

Raw data were processed by DADA2 or deblur to obtain initial amplicon sequence variants (ASVs).

ASVs with an abundance greater than 95 were retained. Species annotation was performed using Quantitative Insights into Microbial Ecology 2 (QIIME2) software.

Inter-group calculation was performed using QIIME2 software to explore phylogenetic relationships among the ASVs and identify differences in dominant species among the samples.

Subsequently, α-diversity and β-diversity analyses were performed based on the output ASVs data.

The α-diversity was estimated by calculating the Chao1 richness estimator, observed species, Shannon diversity index, Simpson index, Faith's PD, Pielou evenness, and Good's coverage, focusing on the richness and evenness of the microbial communities.

The β-diversity was estimated using the Bray-Curtis distance and analysis of similarities (ANOSIM) method.

To compare different species among the samples, pPCoA and NMDS were performed using the vegan R package.

The dominance of bacterial communities among the groups was analyzed using linear discriminant analysis (LDA) effect size (LEfSe), with an LDA score of 2 as the cutoff value.

A cladogram illustrating the relationships of dominant taxa from phylum to genus across different sample communities was plotted using the molecular ecology R package.

Statistical analysis: All the data are expressed as mean ± standard error of the mean (SEM). Statistical analysis was performed using GraphPad Prism (version 8.0.2); one-way analysis of variance (ANOVA) was adopted, followed by Tukey's multiple comparison test. A P-value of less than 0.05 was considered statistically significant.

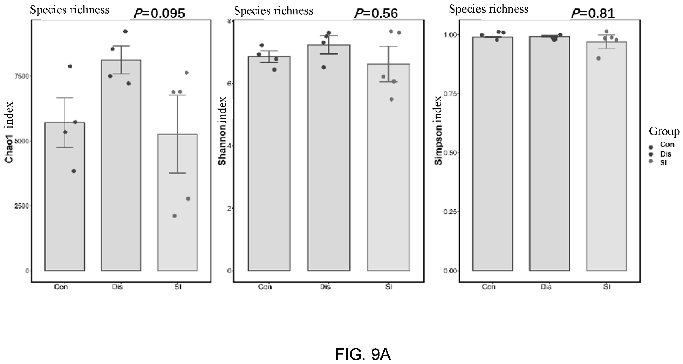

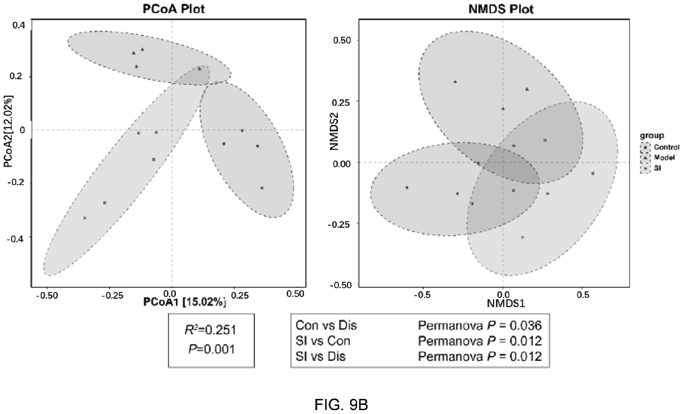

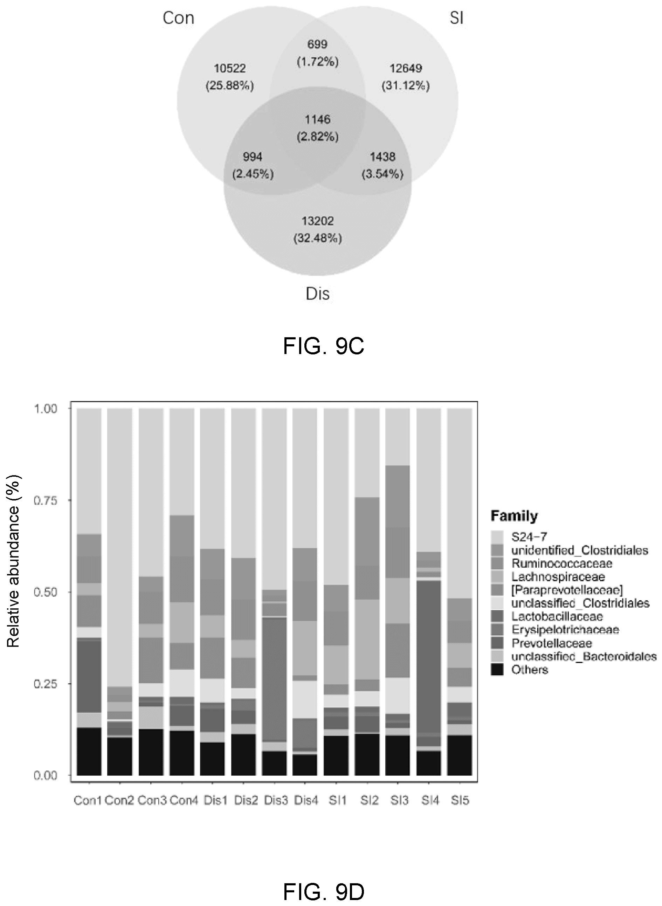

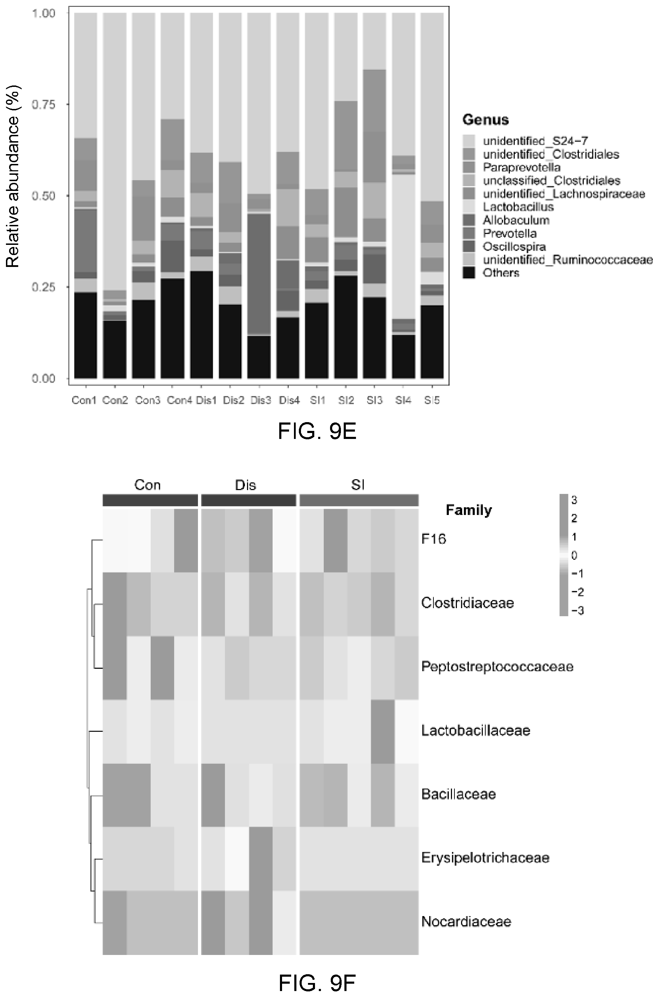

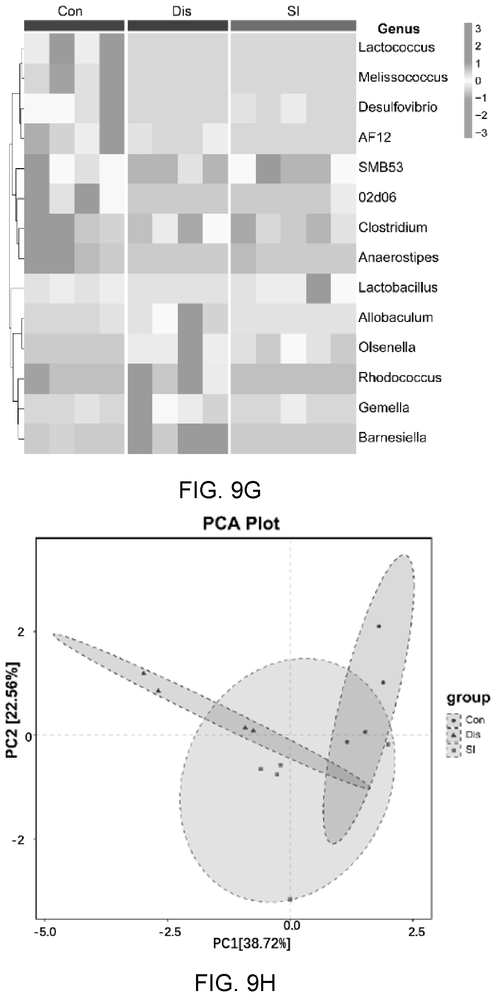

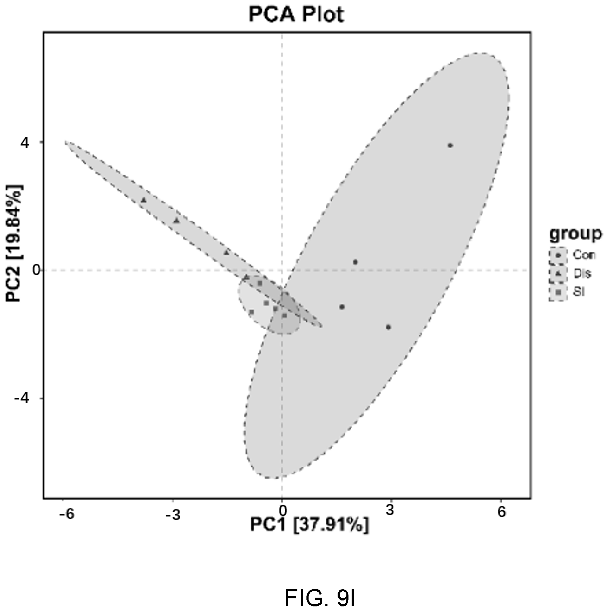

FIG. 9A is a chart illustrating community species richness of gut microbiota evaluated by Chao1 richness estimator, Shannon diversity index, and Simpson index according to some embodiments of the present disclosure. As shown in FIG. 9A, the treatment with the composition derived from the callus culture of snow lotus cells reduces the richness and diversity of the gut microbiota. FIG. 9B is a plot of PCoA and NMDS of the gut microbiota according to some embodiments of the present disclosure. As shown in FIG. 9B, significant differences exist in the microbial composition among the groups. The ANOSIM method indicates that there is a significant difference in the gut microbiota between the model group and the experimental group (p=0.012), and a significant difference also exists in the gut microbiota community structure between the control group and the experimental group (p=0.012), which suggests that the treatment with the composition derived from the callus culture of snow lotus cells changes the heterogeneous community structure. FIG. 9C is a Venn diagram of the gut microbiota of samples from each group according to some embodiments of the present disclosure. As shown in FIG. 9C, differences exist in species distribution among the control group, the model group, and the experimental group. FIGs. 9D-9E are clustering histograms of the gut microbiota according to some embodiments of the present disclosure. FIGs. 9D-9E show specific changes in the bacterial communities, the species composition of each sample, and highlight changes in the gut microbiota at the family and genus levels. FIGs. 9F-9G are graphs illustrating relative abundances of different microorganisms at family and genus levels among groups according to some embodiments of the present disclosure. As shown in FIGs. 9F-9G, at the family level, compared with the control group, F16, Clostridiaceae, and Peptostreptococcaceae are reduced in the model group, while Bacillaceae, Erysipelotrichaceae, and Nocardiaceae are increased in the model group. In contrast, the experimental group treated with the composition derived from the callus culture of snow lotus cells shows an opposite trend, with an increased relative abundance of F16, Clostridiaceae, and Lactobacillaceae, and a reduced relative abundance of Bacillaceae, Erysipelotrichaceae, and Nocardiaceae. At the genus level, relative to the control group, the abundance of Lactococcus, Melissococcus, Desulfovibrio, AF12, SMB53, 02d06, Clostridium, and Anaerostipes is reduced in the model group, while the abundance of Allobaculum, Olsenella, Rhodococcus, Gemella, and Barnesiella is increased in the model group. The treatment with the composition derived from the callus culture of snow lotus cells reduces the relative abundance of SMB53 and Anaerostipes and significantly increases the relative abundance of Lactobacillus, while the relative abundance of Lactobacillus remains unchanged in other groups. FIGs. 9H-9I are PCA plots of different microorganisms at the family and genus levels among groups according to some embodiments of the present disclosure, showing that significant differences exist among the experimental groups.

In summary, administration of the composition derived from the callus culture of snow lotus cells can effectively regulate the abundance of gut microbiota associated with cardiovascular diseases (Barnesiellaceae, Enterococcaceae), metabolic disorders (AF12), and inflammation (Pseudomonadaceae) in the AS model mice.

### EXAMPLE 7: EFFECT OF THE COMPOSITION DERIVED FROM THE CALLUS CULTURE OF SNOW LOTUS CELLS ON CHRONIC LONG-TERM LIVER INJURY

### Materials

### Animals

Blank control mice: C57BL/6 mice, male (4-5 weeks old).

Alcoholic liver disease model mice: C57BL/6 mice, male (4-5 weeks old), continuously fed alcohol.

### Reagents

Dissolving solution: a saline solution containing 0.5% methylcellulose.

High-dose solution of the composition derived from the callus culture of snow lotus cells: 1.1 g of the composition derived from the callus culture of snow lotus cells dissolved in 22 mL of the dissolving solution.

Low-dose solution of the composition derived from the callus culture of snow lotus cells: 2 mL of the high-dose solution of the composition derived from the callus culture of snow lotus cells diluted with 18 mL of the dissolving solution to a final volume of 20 mL.

### Methods

### Experimental group design

The effect of the composition derived from the callus culture of snow lotus cells on ameliorating/treating chronic long-term liver injury was evaluated according to the following experimental design:
Normal group: Blank control mice were administered distilled water daily by oral gavage at a dose of 10 mL/kg.

Model group: Alcoholic liver disease model mice were administered distilled water daily by oral gavage at a dose of 10 mL/kg.

Low-dose group: Alcoholic liver disease model mice were administered the low-dose solution daily by oral gavage at a dose of 50 mg/kg.

High-dose group: Alcoholic liver disease model mice were administered the high-dose solution daily by oral gavage at a dose of 500 mg/kg.

The animals were weighed twice a week, and the dose of the test samples was adjusted according to the body weight. The following indicators were measured monthly: ALT, alkaline phosphatase (ALP), AST, GGT, albumin, malondialdehyde (MDA), glutathione (GSH), triglyceride (TG), and fecal sample collection. Blood was collected from the retro-orbital sinus of the mice after 12 h of fasting with free access to water. At two months of administering the test samples, the animals were sacrificed after 16 h of fasting. After anesthesia with pentobarbital sodium, a median incision was made into the abdomen, blood samples were collected by puncture of the inferior vena cava, liver tissues were separated, and feces, colon, ileum, and jejunum were collected for the detection of various indicators and pathological histological examination.

### Detection and analysis of liver function-related indicators in the first month

The livers and blood of the mice from each group were taken for analysis after 1 month of administration.

Table 7.1 shows the changes in the ALP, ALT, AST, MDA, TG, and GSH levels in the liver of the mice from each group after one month of treatment. Compared with the normal group, the levels of ALP, ALT, AST, MDA, and TG in the liver of the model group are all significantly increased, and the level of GSH is significantly decreased, which indicates successful establishment of a liver injury model. The difference is statistically significant (P<0.05). In contrast, compared with the model group, the levels of ALP, ALT, AST, MDA, and TG in the liver of the high-dose group and the low-dose group are all decreased to varying degrees, and the level of GSH is increased. The difference is statistically significant (P<0.05).

**Table 7.1 Changes in ALP, ALT, AST, MDA, TG, and GSH levels in the liver (x±s)**

| Group | ALP (U/gprot) | ALT (U/gprot) | AST (U/gprot) | MDA (nmol/mgp rot) | TG (mmol/g prot) | GSH (µg/mg prot) |
|---|---|---|---|---|---|---|
| Normal group | 7.88±0.20 | 11.61±2.39 | 16.55±2.03 | 2.11±0.14 | 0.90±0.01 | 14.42±0.22 |
| Model group | 19.20±0.49^{##} | 60.81±5.04^{##} | 27.07±1.92^{#} | 8.31±0.11^{##} | 1.10±0.04^{#} | 11.83±0.53^{#} |
| Low-dose group | 13.52±0.98^{**} | 10.16±1.27^{**} | 18.79±1.97^{*} | 3.27±0.13^{**} | 1.10±0.05 | 13.08±0.68^{*} |
| High-dose group | 14.82±0.91^{*} | 18.42±1.41^{**} | 19.38±2.15 | 5.33±0.11^{**} | 2.25±0.09^{**} | 12.52±0.56 |

| | | | | | | |
|---|---|---|---|---|---|---|
| Note: compared with the normal group: ^{#}P<0.05, ^{##}P<0.01; compared with the model group: *P<0.05, **P<0.01. | | | | | | |

Table 7.2 shows the changes in the GGT, ALB, and GSH levels in the plasma of the mice from each group after one month of treatment. Compared with the normal group, the GGT level in the plasma of the model group is significantly increased, the ALB level does not change significantly, and the GSH level is significantly decreased, which indicates successful establishment of the liver injury model. The difference is statistically significant (P<0.05). Meanwhile, compared with the model group, the GGT level in the plasma of the high-dose group and the low-dose group is significantly decreased, and the GSH level is increased. The difference is statistically significant (P<0.05).

**Table 7.2 Changes in the GGT, ALB, and GSH levels in the plasma (x±s)**

| Group | GGT (U/L) | ALB (g/L) | GSH (µg/mL) |
|---|---|---|---|
| Normal group | 22.02±3.44 | 26.04±0.61 | 15.89±0.18 |
| Model group | 39.54±3.30^{#} | 23.15±0.66^{#} | 13.80±0.35^{#} |
| Low-dose group | 24.67±5.61^{*} | 22.56±0.90 | 14.83±0.82 |
| High-dose group | 21.75±3.08^{*} | 25.61±0.93 | 14.83±0.51 |

| | | | |
|---|---|---|---|
| Note: compared with the normal group: ^{#}P<0.05, ^{##}P<0.01; compared with the model group: *P<0.05, **P<0.01. | | | |

### Detection and analysis of the liver function injury-related indicators in the second month

The liver and the blood of the mice in each group are analyzed after 2 months of administration.

Table 7.3 shows changes in ALP, ALT, AST, MDA, TG, and GSH levels in the liver of the mice in each group after two months of treatment. Compared with the normal group, the levels of ALP, ALT, AST, MDA, and TG in the liver of the model group are all significantly increased, and the level of GSH is significantly decreased, indicating that the chronic long-term liver injury model is successfully established, and the difference is statistically significant (P<0.05). However, compared with the model group, the levels of ALP, ALT, AST, MDA, and TG in the liver of the high-dose group and the low-dose group are all decreased to varying degrees, and the level of GSH is increased, and the difference is statistically significant (P<0.05).

**Table 7.3 Changes in ALP, ALT, AST, MDA, TG, and GSH levels in the liver (x±s)**

| Group | ALP (U/gprot) | ALT (U/gprot) | AST (U/gprot) | MDA (nmol/mg prot) | TG (mmol/gp rot) | GSH (µg/mL) |
|---|---|---|---|---|---|---|
| Normal group | 6.54±0.61 | 68.16±3.23 | 127.70±7.10 | 1.13±0.09 | 0.89±0.06 | 13.98±0.82 |
| Model group | 13.45±0.45^{##} | 282.38±14.41^{##} | 310.22±10.37^{##} | 1.86±0.20^{#} | 0.96±0.03 | 8.54±0.81^{##} |
| Low-dose group | 9.78±0.33^{*} | 186.54±12.58^{**} | 232.84±7.73^{**} | 1.78±0.18 | 0.84±0.04 | 12.76±0.70^{*} |
| High-dose group | 12.80±0.46 | 230.22±8.71^{*} | 257.85±6.85^{*} | 1.74±0.19 | 0.96±0.07 | 10.22±1012 |

| | | | | | | |
|---|---|---|---|---|---|---|
| Note: compared with the normal group: ^{#}P<0.05, ^{##}P<0.01; compared with the model group: *P<0.05, **P<0.01. | | | | | | |

Table 7.4 shows changes in GGT, ALB, and GSH levels in the plasma of the mice in each group after two months of treatment. Compared with the normal group, the level of GGT in the plasma of the model group is significantly increased, the level of ALB is not significantly changed, and the level of GSH is significantly decreased, indicating that the chronic long-term liver injury model is successfully established, and the difference is statistically significant (P<0.05). However, compared with the model group, the level of GGT in the plasma of the high-dose group and the low-dose group is significantly decreased, and the level of GSH is increased, and the difference is statistically significant (P<0.05).

**Table 7.4 Changes in GGT, ALB, ALT, AST, and GSH levels in the plasma (x±s)**

| Group | GGT (U/L) | ALB (g/L) | ALT (U/L) | AST (U/L) | GSH (µg/mL) |
|---|---|---|---|---|---|
| Normal group | 30.25±5.67 | 28.75±2.33 | 15.41±1.80 | 20.77±1.66 | 22.97±0.48 |
| Model group | 106.30±13.16 ^{##} | 33.50±0.67 | 82.76±3.03 ^{##} | 98.50±5.49 ^{##} | 16.43±0.33^{# #} |
| Low-dose group | 48.45±9.72^{*} | 32.32±2.42 | 18.84±1.05 ^{**} | 31.93±7.46 ^{**} | 19.16±0.59^{*} |
| High-dose group | 59.40±8.35^{*} | 33.08±1.04 | 49.97±2.85 ^{**} | 69.89±6.90 ^{**} | 18.25±0.48^{*} |

| | | | | | |
|---|---|---|---|---|---|
| Note: compared with the normal group: ^{#}P<0.05, ^{##}P<0.01; compared with the model group: *P<0.05, **P<0.01. | | | | | |

### Analysis of liver tissue sections

From the middle of the left lobe of the liver of the mice from each group after two months of administration, transverse sections were collected, frozen sections were prepared and subjected to Hematoxylin and Eosin (HE) staining, Oil Red O staining, and IHC staining, followed by microscopic examination.

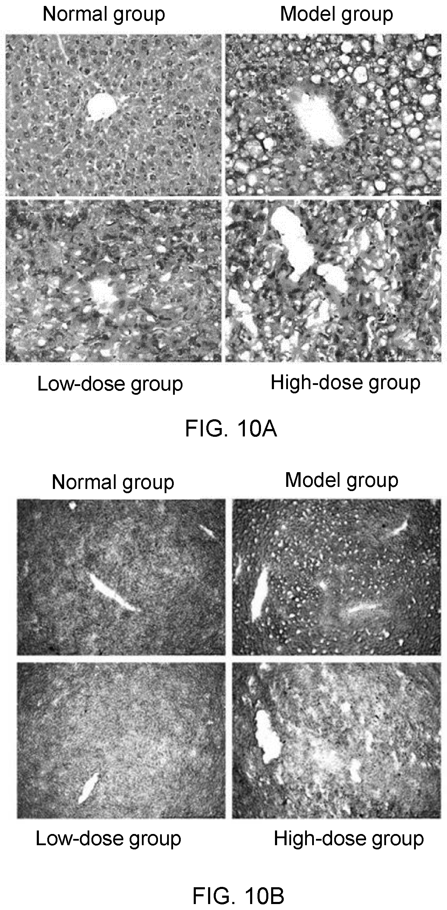

FIG. 10A is an HE staining image of liver sections of mice in different groups according to some embodiments of the present disclosure. Compared with the normal group, severe hepatocellular injury is observed in the model group, with hepatocyte swelling and disorganization around the central vein, and extensive lipid vacuole deposition However, compared with the model group, hepatic structural disorganization is alleviated in the high-dose group and the low-dose group, with mild hepatocyte steatosis and inflammatory infiltration, and hepatic injury is significantly reduced, and the hepatic injury improvement effect in the low-dose group is better.

FIG. 10B is an Oil Red O staining image of liver sections of mice in different groups according to some embodiments of the present disclosure. Compared with the normal group, the model group shows obvious fatty vacuolation and diffuse granular lipid droplets in the liver tissue, indicating histopathological changes consistent with alcoholic liver injury. However, compared with the model group, both the high-dose group and the low-dose group show significantly reduced fatty vacuolation and pathological changes in the liver tissue, and significantly reduced liver injury.

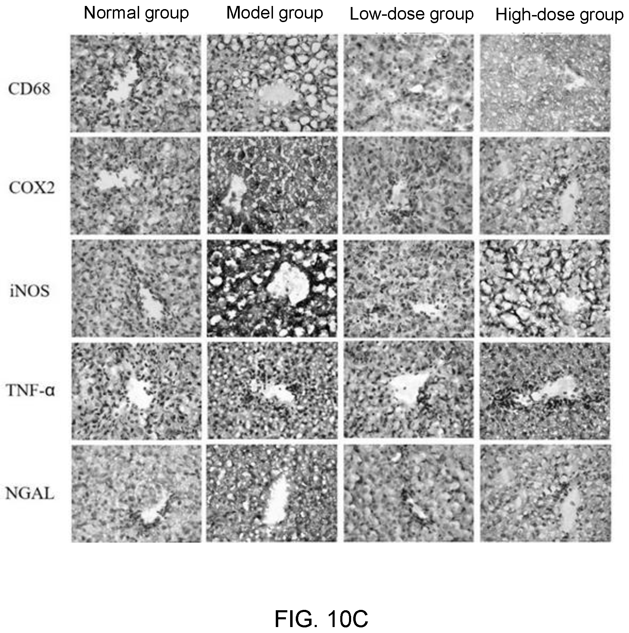

FIG. 10C is an IHC staining image of liver sections of mice in different groups according to some embodiments of the present disclosure. Compared with the normal group, the model group shows significantly increased expression of pro-inflammatory factors (Cluster of Differentiation 68 (CD68), Cyclooxygenase-2 (COX2), Inducible Nitric Oxide Synthase (iNOS), TNF-α, and Neutrophil Gelatinase-Associated Lipocalin (NGAL)) in the liver, along with a marked increase in the number and the size of vacuoles. However, compared with the model group, both the high-dose group and the low-dose group show significantly decreased expression of the pro-inflammatory factors, reduced alcohol-induced lipid metabolic disorders and hepatic steatosis, and significantly reduced hepatic injury.

In summary, the composition derived from the callus culture of snow lotus cells exerts a protective effect on the liver of the chronic long-term liver injury model mice, significantly reducing hepatic injury, with a better effect observed under the low-dose administration.

It should be understood by a person skilled in the art that the above embodiments are merely illustrative of the present disclosure and are not intended to limit the present disclosure. Any modifications, equivalent substitutions, and variations, or the like, made within the spirit and the principle of the present disclosure are intended to be included within the scope of protection of the present disclosure.

Additionally, the present disclosure uses specific terms to describe the embodiments of the present disclosure. As used herein, the terms "an embodiment", "one embodiment", and/or "some embodiments" are intended to indicate a feature, a structure, or a characteristic related to at least one embodiment of the present disclosure. Accordingly, it should be emphasized and noted that the terms "an embodiment", "one embodiment", or "an alternative embodiment" referred to twice or more times at different locations in the present disclosure do not necessarily refer to the same embodiment. Further, certain features, structures, or characteristics in one or more embodiments of the present disclosure may be suitably combined.

In some embodiments, numbers are used to describe quantities of components and properties, and it should be understood that such numbers used in the description of the embodiments are modified in some examples by modifiers "about", "approximate", or "substantially". Unless otherwise indicated, "about", "approximate", or "substantially" indicate that the stated number is allowed to vary by ±20%. Accordingly, in some embodiments, the numerical parameters used in the present disclosure and the claims are all approximate values, and the approximate values may vary according to characteristics required by individual embodiments. In some embodiments, the numerical parameters should take into account specified significant digits and adopt a common method for retaining digits. Although numerical ranges and parameters used in some embodiments of the present disclosure to confirm the breadth of their scope are approximate values, in some specific embodiments, such numerical values are set as precisely as possible within the feasible range.

For each patent, patent application, patent application publication, and other materials cited in the present disclosure, such as articles, books, specifications, publications, documents, or the like, the entirety of their contents is hereby incorporated by reference into the present disclosure. Except for application history documents inconsistent with or conflicting with the content of the present disclosure, and except for documents (currently or subsequently appended to the present disclosure) that limit the broadest scope of the claims of the present disclosure. It should be noted that, if the descriptions, the definitions, and/or the use of terms in auxiliary materials of the present disclosure are inconsistent with or conflict with the content described in the present disclosure, the descriptions, the definitions, and/or the use of terms of the present disclosure shall prevail.

Finally, it should be understood that the embodiments described in the present disclosure are merely illustrative of the principles of the embodiments of the present disclosure. Other modifications may also fall within the scope of the present disclosure. Accordingly, as an example and not as a limitation, alternative configurations of the embodiments of the present disclosure may be considered consistent with the teachings of the present disclosure. Accordingly, the embodiments of the present disclosure are not limited to the embodiments explicitly introduced and described in the present disclosure.

## Claims

1. A composition derived from a callus culture of snow lotus cells, comprising: a) one or more flavonoid compounds in an amount of 7 wt%-20 wt%; b) one or more phenylpropanoid derivatives in an amount of 2.1 wt%-14.1 wt%; c) a carboxylic acid and one or more derivatives thereof, wherein the carboxylic acid and one or more derivatives thereof comprise dicaffeoylquinic acid in an amount of 1.6 wt%-35 wt%; and d) other components.

2. The composition derived from the callus culture of snow lotus cells according to claim 1, wherein the one or more phenylpropanoid derivatives comprise syringin in an amount of 0.3 wt%-5 wt% and chlorogenic acid in an amount of 0.3 wt%-1.3 wt%.

3. The composition derived from the callus culture of snow lotus cells according to claim 1, wherein the dicaffeoylquinic acid comprises 1,5-dicaffeoylquinic acid in an amount of 1.5 wt%-7.7 wt%, and a proportion of the 1,5-dicaffeoylquinic acid in the dicaffeoylquinic acid is 22%-95%.

4. The composition derived from the callus culture of snow lotus cells according to claim 1, wherein a High Performance Liquid Chromatography (HPLC) characteristic chromatogram of the composition at 265 nm comprises 9 characteristic peaks; wherein peak 1 is syringin, peak 2 is chlorogenic acid, peak 4 is 1,4-dicaffeoylquinic acid, peak 5 is 1,5-dicaffeoylquinic acid, peak 6 is 3,5-dicaffeoylquinic acid, peak 7 is dicaffeoylquinic acid malate ester, peak 8 is 1,3,5-tricaffeoylquinic acid malate ester, and peak 9 is 1,3,5-tricaffeoylquinic acid methyl malate ester.

5. The composition derived from the callus culture of snow lotus cells according to claim 4, wherein in the HPLC characteristic chromatogram of the composition at 265 nm, with peak 5 as a reference peak, a relative retention time of each characteristic peak is within a range of ±5% of a specified value as follows: a specified value for peak 1 is 0.369; a specified value for peak 2 is 0.450; a specified value for peak 3 is 0.668; a specified value for peak 4 is 0.918; a specified value for peak 5 is 1.000; a specified value for peak 6 is 1.057; a specified value for peak 7 is 1.140; a specified value for peak 8 is 1.622; and a specified value for peak 9 is 1.645.

6. A method for producing a composition derived from a callus culture of snow lotus cells, comprising:
obtaining a differentiated embryogenic pluripotent stem cell population derived from wild *Saussurea involucrata*; and
performing amplification culture on the differentiated embryogenic pluripotent stem cell population to harvest a culture broth containing the composition;
wherein the differentiated embryogenic pluripotent stem cell population is obtained by a process of controlled differentiation, and the process causes the differentiated embryogenic pluripotent stem cell population to possess the following characteristics: using *in vitro* cells of the wild *Saussurea involucrata* as a control group, differentiation-related genes in the differentiated embryogenic pluripotent stem cell population are expressed at a reduced level, and the differentiation-related genes comprise WOX4, LBD18, WOX5, and PLT4.

7. The method for producing the composition derived from the callus culture of snow lotus cells according to claim 6, wherein the composition comprises: a) one or more flavonoid compounds in an amount of 7 wt%-20 wt%; b) one or more phenylpropanoid derivatives in an amount of 2.1 wt%-14.1 wt%; c) a carboxylic acid and one or more derivatives thereof, wherein the carboxylic acid and one or more derivatives thereof comprise dicaffeoylquinic acid in an amount of 6.8 wt%-8.1 wt%; and d) other components.

8. The method for producing the composition derived from the callus culture of snow lotus cells according to claim 7, wherein the dicaffeoylquinic acid comprises 1,5-dicaffeoylquinic acid in an amount of 1.5 wt%-7.7 wt%, and a proportion of the 1,5-dicaffeoylquinic acid in the dicaffeoylquinic acid is 22%-95%.

9. The method for producing the composition derived from the callus culture of snow lotus cells according to claim 6, wherein expression levels of the WOX4 and the LBD18 in the differentiated embryogenic pluripotent stem cell population are less than 1/10 of expression levels of corresponding genes in the control group; and expression levels of the WOX5 and the PLT4 in the differentiated embryogenic pluripotent stem cell population are less than 1/2 of expression levels of corresponding genes in the control group.

10. The method for producing the composition derived from the callus culture of snow lotus cells according to claim 6, further comprising, during the amplification culture, performing metabolic regulation on the differentiated embryogenic pluripotent stem cell population based on flavonoid metabolic pathway enhancement.

11. The method for producing the composition derived from the callus culture of snow lotus cells according to claim 10, wherein the metabolic regulation causes the differentiated embryogenic pluripotent stem cell population to possess the following characteristics: flavonoid metabolic pathway-related genes in the differentiated embryogenic pluripotent stem cell population are expressed, and the flavonoid metabolic pathway-related genes comprise PAL, C4H, 4CL, CHI, C3H, F5H, and CAD; and, using the *in vitro* cells of the wild *Saussurea involucrata* as the control group, expressions of C3H, F5H, and CAD in the differentiated embryogenic pluripotent stem cell population are increased.

12. The method for producing the composition derived from the callus culture of snow lotus cells according to claim 6, further comprising separating the composition from the culture broth, and preparing an extract from the composition; wherein the extract is prepared by a process comprising:
subjecting the composition to repeated freeze-thawing to obtain a thawed mixture;
performing an extraction treatment on the thawed mixture using an aqueous ethanol solution to obtain a preliminary extract, wherein the extraction treatment includes at least one of ultrasonic extraction or stirring extraction;
separating a solid part and a liquid part of the preliminary extract; and
subjecting the liquid part to a drying process to obtain the extract.

13. Use of the the composition derived from the callus culture of snow lotus cells according to claim 1 in the manufacture of a medicament for atherosclerosis.

14. The use according to claim 13, wherein, compared with a model group with atherosclerosis, formation of atherosclerotic plaques in blood vessels is reduced in a treatment group administered with the composition.

15. The use according to claim 13, wherein, compared with a model group with atherosclerosis, in a treatment group administered with the composition, gut microbiota distribution is regulated, wherein the regulation reduces activation and migration of macrophages, and reduces apoptosis of endothelial cells, thereby reducing the formation of atherosclerotic plaques.

16. The use according to claim 13, wherein the composition is obtained by performing amplification culture on a differentiated embryogenic pluripotent stem cell population derived from wild *Saussurea involucrata*, wherein the differentiated embryogenic pluripotent stem cell population is obtained by a process of controlled differentiation, and the process causes the differentiated embryogenic pluripotent stem cell population to possess the following characteristics: using *in vitro* cells of the wild *Saussurea involucrata* as a control, differentiation-related genes in the differentiated embryogenic pluripotent stem cell population are expressed at a reduced level, and the differentiation-related genes comprise WOX4, LBD18, WOX5, and PLT4.

17. A use of the the composition derived from the callus culture of snow lotus cells according to claim 1 in the manufacture of a medicament for chronic long-term liver injury.

18. The use according to claim 17, wherein, compared with a model group with chronic long-term liver injury, in a treatment group administered with the composition, levels of liver function injury-related indicators are reduced, and the liver function injury-related indicators comprise alanine aminotransferase (ALT), aspartate aminotransferase (AST), gamma-glutamyltransferase (GGT), and alkaline phosphatase (ALP); and in the treatment group, generation of fibrous tissue in liver tissue is reduced, and formation and accumulation of adipose tissue are reduced, thereby reducing formation of alcoholic fatty liver.

19. The use according to claim 17, wherein, compared with a model group with chronic long-term liver injury, in a treatment group administered with the composition, gut microbiota distribution is regulated, wherein the regulation increases a quantity of beneficial bacteria and decreases a quantity of harmful bacteria, thereby reducing a difference between the gut microbiota distribution of the treatment group and gut microbiota distribution of a normal group.

20. The use according to claim 17, wherein the composition is obtained by performing amplification culture on a differentiated embryogenic pluripotent stem cell population derived from wild *Saussurea involucrata*, the differentiated embryogenic pluripotent stem cell population is obtained by a process of controlled differentiation, and the process causes the differentiated embryogenic pluripotent stem cell population to possess the following characteristics: using *in vitro* cells of the wild *Saussurea involucrata* as a control, differentiation-related genes in the differentiated embryogenic pluripotent stem cell population are -expressed at a reduced level, and the differentiation-related genes comprise WOX4, LBD18, WOX5, and PLT4.
